(19) **Europäisches Patentamt**
**European Patent Office**
**Office européen des brevets**

(11) **EP 3 838 286 B1**

(12) **EUROPEAN PATENT SPECIFICATION**

(45) Date of publication and mention
of the grant of the patent:
**15.10.2025   Bulletin 2025/42**

(51) International Patent Classification (IPC):
***A61K 38/08*** (2019.01)        ***A61K 38/10*** (2006.01)
***A61Q 17/00*** (2006.01)

(21) Application number: **19218273.1**

(22) Date of filing: **19.12.2019**

(52) Cooperative Patent Classification (CPC):
**A61K 8/64; A61Q 17/005; C07K 14/43568;**
A61K 38/00; Y02A 50/30

(54) **ANTIMICROBIAL PEPTIDE FROM POLISTES GALLICUS VENOM AND ITS ANALOGS**

ANTIMIKROBIELLES PEPTID AUS POLISTES GALLICUS GIFT UND DESSEN ANALOGA

PEPTIDE ANTIMICROBIEN PROVENANT DU VENIN DE POLISTES GALLICUS ET SES
ANALOGUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.06.2021   Bulletin 2021/25**

(73) Proprietors:
• **Latoxan**
  **26800 Port-Lès-Valence (FR)**
• **CENTRE NATIONAL DE LA RECHERCHE
  SCIENTIFIQUE (CNRS)**
  **75794 Paris cedex 16 (FR)**
• **Universite d'Aix-Marseille (AMU)**
  **13284 Marseille cedex 07 (FR)**

(72) Inventors:
• **MABROUK, Kamel**
  **13170 Les Pennes-Mirabeau (FR)**
• **MARESCA, Marc**
  **13127 Vitrolles (FR)**
• **DE POMYERS, Harold**
  **26260 MARSAZ (FR)**
• **GIGMES, Didier**
  **13190 Allauch (FR)**

(74) Representative: **Barbot, Willy**
  **Simodoro-ip**
  **82, rue Sylvabelle**
  **13006 Marseille (FR)**

(56) References cited:
• **DATABASE UniProt [online] NCBI; 12 January
  2018 (2018-01-12), "MerR family transcriptional
  regulator [Brevundimonas sp. AAP58].",
  XP002799034, Database accession no.
  WP_054111746**
• **CEROVSKY V ET AL: "New potent antimicrobial
  peptides from the venom of Polistinae wasps
  and their analogs", PEPTIDES, ELSEVIER,
  AMSTERDAM, NL, vol. 29, no. 6, 1 June 2008
  (2008-06-01), pages 992 - 1003, XP022646985,
  ISSN: 0196-9781, [retrieved on 20080219], DOI:
  10.1016/J.PEPTIDES.2008.02.007**
• **JI HYEONG BAEK ET AL: "Venom peptides from
  solitary hunting wasps induce feeding disorder
  in lepidopteran larvae", PEPTIDES, ELSEVIER,
  AMSTERDAM, NL, vol. 32, no. 3, 8 December
  2010 (2010-12-08), pages 568 - 572,
  XP028361473, ISSN: 0196-9781, [retrieved on
  20101222], DOI: 10.1016/
  J.PEPTIDES.2010.12.007**
• **MRCIA RENATA MORTARI ET AL:
  "Pharmacological characterization ofvenom: An
  aggressive social wasp widely distributed in the
  Neotropical region", TOXICON, ELMSFORD, NY,
  US, vol. 59, no. 1, 2 November 2011 (2011-11-02),
  pages 163 - 170, XP028437787, ISSN: 0041-0101,
  [retrieved on 20111117], DOI: 10.1016/
  J.TOXICON.2011.11.002**

Remarks:
The complete document including Reference
Table(s) and the Sequence Listing(s) can be
downloaded from the EPO website

Description

FIELD OF THE INVENTION

The present invention relates to a novel family of antimicrobial peptides designated "s" and their synthetic analogs with a unique helical structure isolated from the venom of the social wasp *Polistes gallicus.* These peptides have antimicrobial, anti-inflammatory and anti-proliferative activities. The invention also relates to nucleic acid molecules encoding the peptides, vectors and host cells containing said nucleic acid molecules and methods for producing the peptides. Cosmetic and pharmaceutical compositions comprising the peptides are also part of the invention.

BACKGROUND OF THE INVENTION

Since 1962, only two new classes of antibiotics, oxazolidinones (Linezolid™, Pfizer) and cyclic lipo-peptides (Daptomycin™, Cubist) have come to the market. They are now subject to bacterial resistance too. No novel class of antimicrobial agents has been developed since the 1980s. Thus, there is an urgent need to find new molecules able to: i) fight the wide diversity of resistant pathogen strains and ii) slow down or even impede the development of resistance thanks to new mechanisms of action.

Recently, the search of new drugs and target sites prompted an interest in a group of short, 10-40 amino acid residues, called antimicrobial peptides (AMPs). AMPs represent an ancient defence mechanism that transverses the evolutionary spectrum and remains an effective strategy against invading pathogens. Because of their selectivity for prokaryotic membranes and their membrane-disruptive mechanisms for which microbes have little natural resistance, AMPs are of interest due to their potential as novel antibiotics. Although AMPs are active against bacteria, their sensitivity to proteases and their potential toxicity against human cells prevent their clinical use in vivo.

In recent years, venoms and venom components taken from different insects have shown potential antimicrobial activity.

Mastoparan, the major component of wasp venom is a 14 amino acids in length with an amidated C-terminus, and it is rich in hydrophobic and basic amino acids, resulting in an amphipathic chemical character, adopting an $\alpha$-helical secondary structure under proper conditions. This chemical feature is essential for their biological activities through the cell membrane, and accordingly, they are also called cytolytic peptides. Mastoparan exhibited potent growth inhibition against Gram-positive bacteria but also significant hemolytic activity in human erythrocytes and strong toxicity against mammalian cells, which represent the main limit to its therapeutic use.

Other AMPs found in wasps, such as eumenine-mastoparan, eumenitin, protonectin and paulistine seem to be the most promising molecules as candidate for a therapeutic use due to their lower toxicity against mammalian cells. Particularly, a study by Konno et al. (Peptides, 2006 Nov;27(11):2624-31) showed that eumenitin is effective against Gram-negative bacteria, such as *E. coli* and *P. aeruginosa* at 6$\mu$M and 30 $\mu$M respectively. The same authors highlighted that eumenitin was able to moderately stimulate mast cells degranulation from rat peritoneal mast cells and RBL-2H3 cells. Paulistine, a 21 amino acid peptide extracted from the venom of the social wasp *Polybia paulista* is characterized by a significant antibacterial activity. However, it causes pain, inflammation and chemotaxis of polymorphonuclear leukocytes (Gomes et al., Biochim Biophys Acta. 2014 Jan;1840(1):170-83).

Also, the study "New potent antimicrobial peptides from the venom of Polistinae wasps and their analogs," by Václav Cerovský et al., published in Peptides (2008 Jun;29(6):992-1003), examined the antimicrobial efficacy of mastoparan family peptides. These peptides, isolated from wasps and chemically modified to reduce toxicity, demonstrated strong activity against common pathogens including *E. coli* and *Staphylococcus aureus.* Despite their high antimicrobial activity, the challenges of chemical modifications needed to reduce toxicity complicate their clinical adoption, hindered by issues of in vivo stability and immunogenicity. Building on previous research, the protein comprising 143 amino acids from *Brevundimonas sp. AAP58,* identified in the January 12, 2018 publication with sequence WP_054111746.1, shows potential for influencing microbial responses. This protein belongs to the MerR family transcriptional regulator and is characterized by a Helix-Turn-Helix DNA binding domain of putative MIrA-like transcription regulators.

Despite the clear potential of AMPs, only very few AMPs such as polymyxins and gramicidins are being used clinically. The use of AMPs is mainly limited by: i) the hemotoxicity and or systemic toxicities, ii) the poor *in vivo* stability and the immunogenicity which remain major problems when considering systemic therapeutic applications (Yount and Yeaman, Pharmacol. Rev., 2003; 55, 27-55).

There is therefore a need for a method allowing to overcome these shortcomings, and in particular for identifying and selecting AMPs suitable to treat microbial infections.

The serious problems caused by drug resistant bacteria have created an urgent need for the development of alternative therapeutics. In this respect, AMPs are considered as promising antimicrobial agents for producing new generation antibiotics. Additionally, atomic level structures of AMPs are prerequisite information for the generation of improved peptide antibiotic candidates. Although there are several obstacles to be overcome for clinical applications, natural and synthetic AMPs are still attractive sources to the pharmaceutical companies. In terms of production cost, the use of those peptides would be advantageous in the pharmaceutical field.

## SUMMARY OF THE INVENTION

The inventors have now identified a novel family of peptides isolated and derived form peptides found the venom of the social wasp *Polistes gallicus.* The peptides of the invention can furthermore be used as antimicrobial, anti-proloferative or anti-inflammatory agent. The peptides of the invention can furthermore be used for treating conditions related to bacterial or fungus infection.

Consequently, the present invention relates to an engineered peptide comprising or consisting of the amino acid sequence $ILX_1X_2X_3X_4X_5LLX_6NL$ (SEQ ID NO:1), wherein:

$X_1$ is absent or is an amino acid selected from the group consisting of S, K and W,
$X_2$ is absent or is an amino acid selected from the group consisting of A and L,
$X_3$ is an amino acid selected from the group consisting of I and W,
$X_4$ is an amino acid selected from the group consisting of L and K,
$X_5$ is an amino acid selected from the group consisting of G or K, and
$X_6$ is an amino acid selected from the group consisting of K and R,

and the functional derivatives or salts thereof, wherein the functional derivatives are selected from diastereomeric peptides, all-D-peptides, retropeptides and peptides in which the sequence of SEQ ID NO:1 is shortened by 1 to 4 amino acids at the N-terminal and /or C-terminal end with the proviso that the peptide has a size comprised between 7 and 10 amino acid .

In one embodiment, the engineered polypeptide of the invention is as in claim 4 or 5.The present invention also relates to a nucleic acid encoding for an engineered peptide of the invention, an expression cassette comprising it, an expression vector comprising it or a host cell transformed with the nucleic acid encoding for an engineered peptide of the invention.

The present invention also relates to a cosmetic or pharmaceutical composition comprising at least one engineered peptide of the invention, and a cosmetically or pharmaceutically acceptable support and/or excipient.

Since the engineered peptide of the invention has antimicrobial or antifungal activity, it can be used as medicament, and in particular for use in the treatment of a condition caused by a bacterium or a fungus.

The present invention also relates to the *in vitro* use of an engineered peptide of the invention as disinfectant or preservative agent.

Finally, the present invention relates to a medical device or implant comprising a body having at least one surface coated with or including an engineered peptide of the invention.

The present invention and its preferred embodiments are described in further detail below.

## BRIEF DESCRIPTION OF THE DRAWINGS

Figure 1 shows the Purification of gallicune peptides from *Polistes gallicus* venom. In particular, figure 1-A illustrates reverse phase (RP)-HPLC fractionation of *Polistes gallicus* venom. Figure 1-B illustrates the region of RP-HPLC chromatogram containing the three antimicrobial peptides of *Polistes gallicus* venom.

Figure 2 shows the RP-HPLC chromatogram of purified gallicune peptides named Dominulin A, Dominulin B and Gallicus-CP I1L12 respectively.

Figure 3: shows the hemolytic activity of Gallicus-CP peptide I1L12 (A) and its functional derivatives LTX-gallicune-2 R1W8 (○) and LTX-gallicune-1 I1N11 (■) against human erythrocytes.

Figure 4: shows concentration-response curves of the LTX-gallicune-1 peptides I1N11, I1K10 and their respective functional derivatives I1(d)N11(d), (I1N11)d, I1(d)K10(d) and (I1K10)d in the growth inhibition against MIA PaCa-2 tumor cells. The *in vitro* cytotoxicity was determined using the resazurin assay.

Figure 5: shows the inhibition effect of the peptides I1N11 and I1K10 and their respective functional derivatives I1(d)N11(d), (I1N11)d, I1(d)K10(d) and (I1K10)d on LPS stimulation. Polymyxin B (PMB) was used as positive control..

Figure 6: shows the dose-effect curves and the permeabilization kinetics of the cytoplasmic membranes using the peptides Gallicus-CP I1L12 (A), LTX-gallicune-2-R1W8 (○) and LTX-gallicune-1 I1N11 (■) on the bacteria *Bacillus subtilis* (A), *Staphylococcus aureus* (B), *Escherichia coli* (C) and *Pseudomonas aeruginosa* (D). The cationic detergent cetyltrimethylammonium bromide (CTAB) (+) is used as positive control.

Figure 7: shows resistance acquisition during serial passaging of *P. aeruginosa* in the presence of sub-MIC levels of LTX-gallicune-1 I1N11 (■). Imipenem (Ж) and gemifloxacin (-) are antibiotics.

Figure 8: shows resistance acquisition during serial passaging of *B. subtilis* in the presence of sub-MIC levels of LTX-gallicune-2 R1W8 (○). Mupirocin (-) is an antibiotic.

## DETAILED DESCRIPTION OF THE INVENTION

The composition of *Polistes gallicus* venom has never been described or disclosed to date in any other study. Surprisingly, the inventors isolated from *Polistes gallicus* venom one peptide having antimicrobial activity toward bacteria and yeasts. This peptide, called the Gallicus Chemotactic Peptide (Gallicus-CP $I_1L_{12}$) is a peptide of 12 amino acids in length and has the sequence ILSAILGLLKNL (SEQ ID NO: 2). The expressions I1L12 and $I_1L_{12}$ are similar and can be used interchangeably; similar terminology can be used for all the engineered peptides of the invention.

Gallicus-CP $I_1L_{12}$ peptide exhibits antimicrobial activity against Gram-positive and Gram-negative bacteria with a minimal inhibitory concentration (MIC) ranging from 1.56 to 100 $\mu$M depending on bacterial strains. Nevertheless, despite this interesting antibacterial activity, the hemolytic and cytotoxic activities of Gallicus-CP $I_1L_{12}$ peptide are too high to consider its use as a drug.

In a surprising manner, the inventors found that the substitution of one or more amino acids of Gallicus-CP $I_1L_{12}$ peptide by amino acids with positive charged side chain or with hydrophobic side chain reduces hemolytic and cytotoxic activities while preserving antimicrobial activity.

In a first aspect, the present invention relates to an engineered peptide comprising or consisting of the amino acid sequence $ILX_1X_2X_3X_4X_5LLX_6NL$ (SEQ ID NO:1), wherein:

$X_1$ is absent or is an amino acid selected from the group consisting of S, K and W,
$X_2$ is absent or is an amino acid selected from the group consisting of A and L,
$X_3$ is an amino acid selected from the group consisting of I and W,
$X_4$ is an amino acid selected from the group consisting of L and K,
$X_5$ is an amino acid selected from the group consisting of G or K, and
$X_6$ is an amino acid selected from the group consisting of K and R,

and the functional derivatives or salts thereof, wherein the functional derivatives are selected from diastereomeric peptides, all-D-peptides, retropeptides, and peptides in which the sequence of SEQ ID NO:1 is shortened by 1 to 4 amino acids at the N-terminal and /or C-terminal end with the proviso that the peptide has a size comprised between 7 and 10 amino acids.

This consensus sequence corresponds to the sequence alignment based on the sequence of Gallicus-CP $I_1L_{12}$ peptide isolated from *Polistes gallicus* venom and the sequences of all the functional derivatives designed by the inventors that were shown to exhibit biological properties.

By "engineered peptide" is meant a non-naturally occurring peptide that has been designed and/or produced by the hand of man. In all embodiments, the engineered peptide of the invention derived from the isolated Gallicus Chemotactic Peptide (Gallicus-CP) $I_1L_{12}$ and comprises an amino acid sequence derived from the sequence ILSAILGLLKNL (SEQ ID NO: 2). Modifications of peptide structures may involve backbone and/or side chain modifications such as incorporation of unnatural amino acids (D-amino acids, $\beta$-amino acids, rare amino acids), of N-substituted glycines, chemical modification of terminal ends of peptides (amidation, esterification, acylation, acetylation), shortening of the native sequence, modifications of their amphipathic character, cyclization, lipidation, etc. All modifications made to the peptide aim to improve interactions with the bacterial membrane and thus, in general, antimicrobial activity and stability, while keeping their hemolytic activity as low as possible.

By "functional derivatives" is meant , for example, retro-peptides, By "functional derivatives" is also meant peptides according to the invention whose sequence is shortened by 1, 2, 3, or 4, amino acids at the N-terminal and/or C-terminal end, preferably by 1 or 2 amino acids at the C-terminal end.

One strategy to modify and improve the antimicrobial properties is to change from L- to D-amino acids. D-Amino acids are very rare in nature and their incorporation changes the side chain and the backbone properties of the peptide. Structural changes due to the insertion of D-amino acids within peptides must not occur at the expense of their biological activity, in particular their antimicrobial activity.

In some embodiments, an engineered peptide has an amino acid sequence that includes D-form amino acids. The replacement of L-amino acids by their respective D-enantiomers can occur at one or more position within the peptide and/or at its N- and/or C-terminal end. Peptide composed of both L-and D-amino acids is called "diastereomeric peptide" wherein one or more of the L-amino acids have been converted to the opposite configuration, i.e. D-amino acid. When all L-amino acids of the peptide are replaced by their respective D-enantiomers, the peptide is called "all-D-amino acid peptide", inverso-peptide or enantio.

N-substituted glycines determine a relocation of the side chain from the $\alpha$-carbon to the nitrogen.

By "retro-peptide" is meant peptide consisting of the same sequence of L amino acids but in reverse order. In retro-peptide, the amino acid in N-terminal corresponds to the amino acid in C-terminal of the parent peptide. In other words, the retro-peptide has a reversed peptide sequence from the N-to C-terminus from that of the parent peptide. The retro-peptide of ILSAILGLLKNL (SEQ ID NO: 2) is LNKLLGLIASLI (SEQ ID NO:3).

In a preferred embodiment, the engineered peptide of the invention comprises or consists of the amino acid sequence wherein $X_1$ is an amino acid selected from the group consisting of S and K, $X_2$ is A, $X_3$ is I, $X_4$ is an amino acid selected from the group consisting of L and K, $X_5$ is an amino acid selected from the group consisting of G and K, $X_6$ is K.

In a another preferred embodiment, the engineered peptide of the invention comprises or consists of the amino acid sequence wherein $X_1$ is W, $X_2$ is L, $X_3$ is W, $X_4$ is L, $X_5$ is G and $X_6$ is R.

According to a preferred embodiment, the engineered peptide of the invention consists of a sequence selected from the Table 1.

**Table 1: Gallicus-CP $I_1L_{12}$ peptide and its derivatives**

| Peptides Name | Amino acid sequences | Sequence ID | Molecular weight (Da) | Length | Net Charge |
|---|---|---|---|---|---|
| Gallicus-CP($I_1L_{12}$) | ILSAILGLLKNL | SEQ ID NO : 2 | 1266 | 12 | +1 |
| $I_1L_9$ | ILSAILGLL | SEQ ID NO : 4 | 911 | 9 | +1 |
| $I_1K_{10}$ | ILSAILGLLK | SEQ ID NO : 5 | 1039 | 10 | +2 |
| $K_{10}I_1$ | KLLGLIASLI | SEQ ID NO : 6 | 1039 | 10 | +2 |
| $L_2K_{10}$ | LSAILGLLK | SEQ ID NO : 7 | 926 | 9 | +2 |
| $L_2L_9$ | LSAILGLL | SEQ ID NO : 8 | 798 | 8 | +1 |
| $L_8L_{12}$ | LLKNL | SEQ ID NO : 9 | 599 | 5 | +2 |
| $L_6L_{12}$ | LGLLKNL | SEQ ID NO : 10 | 769 | 7 | +2 |
| $S_3L_{12}$ | SAILGLLKNL | SEQ ID NO : 11 | 1040 | 10 | +2 |
| $S_3L_9$ | SAILGLL | SEQ ID NO : 12 | 685 | 7 | +1 |
| $L_{12}I_1$ | LNKLLGLIASLI | SEQ ID NO : 3 | 1266 | 12 | +1 |
| $(i_1l_{12})$enantio | ilsailgllknl | SEQ ID NO : 13 | 1266 | 12 | +2 |
| $K_1I_8$ | KLLGLILI | SEQ ID NO : 14 | 882 | 8 | +2 |

The upper case letter indicates a normal L-type amino acid. The lower case letter indicates a D-type amino acid. Enantio indicates an all D-amino acids peptide.

In this preferred embodiment, the engineered peptide of the invention has a net charge comprised between +1 and +2.

In this preferred embodiment, the engineered peptide of the invention has molecular weight measured by mass spectrometry comprised between 550 and 1300 Da, preferably between 650 and 1050 Da.

Studying structure-activity relationships using synthetic peptides designed from Gallicus-CP $I_1L_{12}$ sequence enabled the inventors to conceive two novel antimicrobial peptides (AMPs) named LTX-gallicune-1(I1N11) and LTX-gallicune-2 (R1W8) with similar or increased inhibitory activity against bacteria and fungi and reduced cytotoxicity as compared to Gallicus-CP $I_1L_{12}$ peptide.

Preferably, the engineered peptide of the invention exhibits no or weak cytolytic activity on mammalian cell, either animal or human. In particular, the engineered peptide of the invention may have a median lethal concentration (LC50) of more than 10 $\mu$M for cells, preferably more than 20, 40, 60, 80, 100, 200, 500 $\mu$M. LC50 corresponds to the peptide concentration inducing hemolysis of 50% of the cells. The LC50 value may be obtained for example on rat, dog, rabbit, pig, horse, cat or human cells, preferably on human cells. In a more preferred embodiment, the engineered peptide of the invention may have a minimal hemotoxic concentration (MHC50) of more than 10 $\mu$M for erythrocytes, preferably more than 20, 40, 60, 80, 100, 200, 500 $\mu$M. MHC50 corresponds to the minimum peptide concentration inducing 50% lysis of human red blood cells. The MHC50 value may be obtained for example on rat, dog, rabbit, pig, horse, cat or human erythrocytes, preferably on human erythrocytes.

Preferably, the engineered peptide of the invention exhibits no or weak cytotoxic activity on mammalian cells, either animal or human. In particular, the engineered peptide of the invention may have a half maximal inhibitory concentration (IC50) of more than 300 $\mu$M, more than 400 $\mu$M, more than 500 $\mu$M, more than 600 $\mu$M, more than 700 $\mu$M, more than 800 $\mu$M. The IC50 corresponds to the peptide concentration causing a reduction of 50% of cell viability. The IC50 value may be obtained for example on rat, dog, rabbit, pig, cat or human cells. Mammalian cells may be primary cells or cells derived from cell lines.

According to another preferred embodiment, the engineered peptide of the invention consists of a sequence selected from the group consisting of SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO:24, SEQ ID NO: 25, SEQ ID NO: 26, SEQ ID NO: 27 and SEQ ID NO: 28.

**Table 2: LTX-gallicune-1 $I_1N_{11}$ and its derivatives**

| Peptides Name | Amino acid sequences | Sequence ID | Molecular weight (Da) | Length | Net Charge |
|---|---|---|---|---|---|
| LTX-gallicune-1 ($I_1N_{11}$) | ILKAIKKLLKN | SEQ ID NO :15 | 1281 | 11 | +4 |
| LTX-gallicune-1(i1D-n11D) | iLKAIKKLLKn | SEQ ID NO :16 | 1281 | 11 | +4 |
| LTX-D (gallicune-1) | ilkaikkllkn | SEQ ID NO :17 | 1281 | 11 | +4 |
| $I_1L_9$* | ILKAIKKLL | SEQ ID NO :18 | 1039 | 9 | +3 |
| $I_1K_7$* | ILKAIKK | SEQ ID NO :19 | 813 | 7 | +4 |
| $L_2L_9$* | LKAIKKLL | SEQ ID NO :20 | 926 | 8 | +4 |
| $L_2k_{10}$* | LKAIKKLLk | SEQ ID NO :21 | 1054 | 8 | +5 |
| $K_3N_{11}$* | KAIKKLLKN | SEQ ID NO :22 | 1055 | 9 | +5 |
| Ac $I_1N_{10}$* | Ac-IKAIKKLLKN | SEQ ID NO :23 | 1210 | 10 | +1 |
| $I_1K_{10}$* | ILKAIKKLLK | SEQ ID NO :24 | 1167 | 10 | +1 |
| $i_1k_{10}$* | iLKAIKKLLk | SEQ ID NO :25 | 1167 | 10 | +5 |
| ($i_1k_{10}$*) enantio | ilkaikkllk | SEQ ID NO :26 | 1167 | 10 | +5 |
| LTX-gallicune-2 ($R_1W_8$) | RLLGLWLW | SEQ ID NO :27 | 1056 | 8 | +1 |
| LTX-gallicune-2 ($r_1W_8$) | rLLGLWLW | SEQ ID NO :28 | 1056 | 8 | +1 |

*= peptide derived from LTX-gallicune-1 ($I_1N_{11}$). The upper case letter indicates a normal L-type amino acid. The lower case letter indicates a D-type amino acid. Enantio indicates an all D-amino acids peptide.

In this preferred embodiment, the engineered peptide of the invention has a net charge comprised between +1 and +5. In another preferred embodiment, the engineered peptide of the invention has a net charge of +4 or +5. In another preferred embodiment, the engineered peptide of the invention has a net charge of +1.

In this preferred embodiment, the engineered peptide of the invention has molecular weight measured by mass spectrometry comprised between 800 and 1300 Da, preferably between 900 and 1200 Da, and more preferably between 1000 and 1100 Da.

In a preferred embodiment, the engineered peptide of the invention consists of the amino acid sequence selected from the group consisting of sequences of SEQ ID NO: 15 to 28, and more preferably from the group consisting of sequences of SEQ ID NO: 15, 16, 17, 24, 25, 26 and 27.The engineered peptide of the invention may be linear or cyclized. The term "cyclized," as used herein, refers to a reaction in which one part of the engineered peptide of the invention becomes linked to another part of the engineered peptide to form a closed ring, such as by forming a disulfide bond, a thioether bond, a thioesther bond, an amide bond, a sulfonamide, a lactam bond, a triazole ring, a selenoether bond, a diselenide bond, or an olefin bond. Such intramolecular bond occurs between two non-contiguous amino acid residues of the engineered peptide.

In a second aspect, the present invention relates to a nucleic acid encoding for an engineered peptide as defined above.

By "nucleic acid" is meant any molecule based on DNA or RNA. These may be synthetic or semi-synthetic, recombinant molecules, possibly amplified or cloned into vectors, chemically modified, comprising non-natural bases or modified nucleotides comprising for example a modified bond, a modified purine or pyrimidine base, or a modified sugar.

The nucleic acid of the invention may be in the form of DNA and/or RNA, single stranded or double stranded. According to a preferred embodiment, the nucleic acid is an isolated DNA molecule, synthesized by recombinant techniques well known to one of skill in the art.

The nucleic acid of the invention may be deduced from the sequence of the peptide according to the invention and codon usage may be adapted according to the host cell in which the nucleic acid shall be transcribed. These steps may be carried out according to methods well known to one of skill in the art and some of which are described in the reference manual Sambrook *et al.* (Sambrook et al.,2001, Molecular Cloning: a laboratory manual, Third Edition Cold Spring Harbor).

In a third aspect, the present invention relates to an expression cassette comprising a nucleic acid as defined above.

The present invention further relates to an expression cassette comprising a nucleic acid of the invention operably linked to the sequences required for its expression. In particular, the nucleic acid may be under the control of a promoter allowing its expression in a host cell. Generally, an expression cassette is constituted of or comprises a promoter allowing initiation of transcription, a nucleic acid of the invention, and a transcription terminator. By "expression cassette" is meant a nucleic acid construct comprising a coding region and a regulatory region, operably linked. The expression "operably linked" indicates that the elements are combined in such a way that the expression of the coding sequence (the gene of interest) and/or the targeting of the encoded peptide are under the control of the transcriptional promoter and/or signal peptide. Typically, the promoter sequence is placed upstream of the gene of interest, at a distance therefrom, which is compatible with the control of expression. Likewise, the sequence of the signal peptide is generally fused upstream of the sequence of the gene of interest, and in the same reading frame with the latter, and downstream of any promoter. Spacer sequences may be present, between the regulatory elements and the gene, as long as they do not prevent expression and/or targeting. In a preferred embodiment, said expression cassette comprises at least one "enhancer" activating sequence operably linked to the promoter.

In a fourth aspect, the present invention relates to an expression vector comprising a nucleic acid or an expression cassette as defined above.

By "expression vector" is meant extra chromosomal element that enables a particular nucleic acid sequence to be expressed in a host cell. The vector may be a DNA or an RNA, circular or not, single- or double-stranded. Advantageously it is selected from the group consisting of a plasmid, a phage, a phagemid, a virus, a cosmid and an artificial chromosome.

Advantageously, the expression vector comprises regulatory elements allowing the expression of the nucleic acid according to the invention. These elements may contain for example transcriptional promoters, transcriptional activators, terminator sequences, initiation and termination codons. The methods for selecting said elements according to the host cell in which expression is desired, are well known to one of skill in the art.

The expression vector may also contain elements enabling its selection in the host cell such as, for example, an antibiotic resistance gene or a selectable gene providing complementation of the respective gene deleted from the host cell genome. Such elements are well known to one of skill in the art and extensively described in the literature.

When the host cell to be transformed is a plant cell, the expression vector is preferably a plant vector. Examples of plant vectors are described in the literature, including in particular the T-DNA plasmids of A. tumefaciens pBIN19, pPZP100, the pCAMBIA series (Lan-Ying Lee and Stanton B. Gelvin, T-DNA Binary Vectors and Systems Plant Physiol. 2008 Feb; 146(2): 325-332.). The expression vectors of the invention may additionally comprise an origin of replication and/or a selectable marker gene and/or a plant recombination sequence.

The expression vectors may be constructed by the classical techniques of molecular biology, well known to one of skill in the art.

In a fifth aspect, the present invention relates to a host cell comprising a nucleic acid, an expression cassette or an expression vector as defined above.

According to one embodiment, the host cell is a microorganism, preferably a bacterium or a yeast.

According to another embodiment, the host cell is an animal cell, for example a mammalian cell such as COS or CHO cells (US 4,889,803 ; US 5,047,335 ). In a particular embodiment, the cell is non-human and non-embryonic.

According to yet another embodiment, the host cell is a plant cell. The term "plant cell" as employed herein refers to any cell coming from a plant and which may constitute undifferentiated tissues such as calluses, and differentiated tissues such as embryos, plant parts, plants or seeds.

In a sixth aspect, the present invention relates to a cosmetic composition comprising at least one engineered peptide as defined above, and a cosmetically acceptable support and/or excipient.

In a seventh aspect, the present invention relates to a pharmaceutical composition comprising at least one engineered peptide as defined above, and a pharmaceutically acceptable support and/or excipient.

In particular embodiment, the pharmaceutical or cosmetic composition may comprise, or consist of, 1, 2, 3, 4 or 5 engineered peptides according to the invention and a pharmaceutically or cosmetically acceptable support and/or excipient.

By "cosmetically or pharmaceutically acceptable support and/or excipient" is meant that the compositions or support and/or excipient thereof are compatible with the tissues to which it will be applied without undue toxicity, incompatibility, instability, allergic response, and the like. When appropriate, compositions of the invention may comprise any ingredient conventionally used in the fields under consideration, and particularly in cosmetics and dermatology.

Specific examples of excipient include, but is not limited to, moisturizers and hydration agents, penetration agents, emulsifiers, natural or synthetic oil solutions, chelating agents, solvents, surfactants, gelling agents, emollients, fragrances, darkening or lightening agents, glitter, humectants, fillers, thickeners, waxes, odor absorbers, dyestuffs, coloring agents, powders such as mica or minerals, viscosity-controlling agents and water, powders, amino acids, polyamino acids and a salt thereof, sugar alcohol and alkylene oxide adducts thereof, lower alcohols, animal and plant extracts, nucleic acids, vitamins, enzymes, anti-inflammatory agents, additional antimicrobial agents such as antibiotics, preservatives, antioxidants, moisturizer, thickener, viscosity modifier, UV absorbers, adiaphoretics, pigments, dyes, flavors, pH adjusters, pearly sheen agents, wetting agents and the like. Lists of these excipients can be found in the patent application US 2016/0354294 A1. These are mere examples and components other than these can be added.

The form of the cosmetic composition of the present invention is not particularly limited, and may take any form such as lotion, serum, suspension, emulsion, paste, cream, foam, ointment, gel, solid, powder and the like, spray or aerosol, tablet.

The compositions of the invention are suitable for local delivery. By "local delivery" is meant that the site of action is at, or near to the site of application of the composition.

When formulated for topical delivery, the cosmetic composition of the invention may be formulated as cream, foam, a gel, a lotion or an ointment. Topical delivery means may include transdermal delivery (e.g. via an adhesive patch, iontophoresis or ultrasound device).

The pharmaceutically acceptable excipients and supports that can be used in the composition according to the invention are well known to one of skill in the art (Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed. , Pharmaceutical Press[2000]) and comprise in particular physiological saline solutions and phosphate buffers. As an example of pharmaceutically support and/or excipient, and without undue limitation, one can cite carriers, such as, but not limited to an albumin, preferably a human serum albumin or a recombinant human albumin, a non-reducing di- or tri-saccharide, a nonionic surfactant.

The pharmaceutical composition according to the invention may be suitable for local or systemic administration, in particular for oral, sublingual, cutaneous, subcutaneous, intramuscular, intravenous, intraperitoneal, topical, intra-tracheal, intranasal, transdermal, rectal, intraocular or intra-auricular administration. Preferably, the pharmaceutical composition according to the invention is suitable for cutaneous, oral, topical, intramuscular, intravenous, transdermal or subcutaneous administration. According to a particular embodiment, the pharmaceutical composition according to the invention is suitable for topical administration. The pharmaceutical composition according to the invention may be in the form of tablets, capsules, soft capsules, granulates, suspensions, emulsions, solutions, gels, pastes, ointments, creams, plasters, potions, suppositories, enemas, injectables, implants, patches, sprays or aerosols.

The compositions of the invention may be in the form of controlled-release or sustained-release compositions, wherein the engineered peptide of the invention along with additional active agents are encapsulated or otherwise contained within a material, such that they are released onto the skin or affected area in a controlled manner over time. The compositions of the invention may be contained within or on matrixes, liposomes, vesicles, microcapsules, microspheres and the like, or within or on a solid particulate material.

The composition of the invention contains an amount of engineered peptide effective for antimicrobial action. Generally, the composition of the invention contains from about 0.5 percent (wt./vol.) to about 30 percent engineered peptide. In certain embodiments, the composition contains from about 1 percent to about 10 percent engineered peptide, such as about 3 percent, about 5 percent, about 7 percent, or about 9 percent of engineered peptide according to the invention.

According to one embodiment, the composition according to the invention comprises from about 0.1 to about 500 mg of engineered peptide of the invention. Preferably, the composition according to the invention comprises from about 1 to about 100 mg, and more preferably from about 5 to about 50 mg of engineered peptide according to the invention.

According to another embodiment, the composition according to the invention contains from about 0.01 to about 100 mg/kg, preferably from about 0.1 to 10 mg/kg of engineered peptide according to the invention.

In an eighth aspect, the present invention relates to an engineered peptide as defined above for use as medicament, in particular as medicament for treating a microbial infection, namely an infection due to a bacterium or a fungus.

In a ninth aspect, the present invention relates to an engineered peptide as defined above for use in the treatment of a condition caused by a bacterium or a fungus.

The condition caused by a bacterium may be due to Gram-negative bacteria. In particular, Gram-negative bacteria may be selected from the group consisting of Escherichia coli and bacteria from the genus Pseudomonas, Salmonella, Acinetobacter or Klebsiella. Preferably, Gram-negative bacteria are selected from the group consisting of *Escherichia coli, Pseudomonas aeruginosa, Salmonella enterica, Acinetobacter baumannii* and *Klebsiella pneumoniae.*

The condition caused by a bacterium may be due to Gram-positive bacteria. In particular, Gram-positive bacteria may be selected from the group consisting of bacteria from the genus *Staphylococcus, Streptococcus, Bacillus, Listeria* or *Enterococcus.* Preferably, Gram-positive bacteria are selected from the group consisting of *Staphylococcus aureus,*

*Streptococcus pyogenes, Bacillus subtilis, Listeria ivanovii* and *Enterococcus faecalis.*

The engineered peptide of the invention may be useful for inhibiting such bacteria to ameliorate microbial infection(s), or reduce the likelihood of microbial infection(s), including: bacteremia, pneumonia, meningitis, osteomyelitis, endocarditis, dental caries, periodontal disease, sinusitis, rhinitis, pink eye, urinary tract infections, tetanus, gangrene, colitis, acute gastroenteritis, impetigo, acne, acne posacue, wound infections, burn infections, fascitis, bronchitis, and a variety of abscesses, nosocomial infections, and opportunistic infections.

In a preferred embodiment, the invention relates to the engineered peptide of the invention for use in the treatment of acne. In this particular embodiment, the composition comprises an effective amount of all D-type engineered peptide i1n11 of SEQ ID NO: 17 as active agent for the treatment of acne.

The condition may also be due to a fungus. In particular, the fungus may be from the genus Candida or Aspergillus. For example, the fungus may be selected from the group consisting of *Candida albicans* and *Candida parapsilosis.*

The engineered peptide of the invention may also be effective for inhibiting the growth and survival of fungal organisms such as dermatophytes (e.g.,*Microsporium spp.* such as *Microsporum canis, Microsporum audouinii,* and *Microsporum gypseum;* and *Trichophyton spp.,* such as *T. rubrum, T. mentagrophytes, T. trichophyton, T. schoenleinii,* and *T. tonsurans*)*, Acremonium spp., Fusarium oxysporum, Scopulariopsis brevicaulis, Onychocola canadensis, Scytalidium dimidiatum*; yeasts (*e.g.,Candida albicans, C. Tropicalis,* or other Candida species, and *Saccharomyces cerevisiae*)*, Torulopsis glabrata, Epidermophyton floccosum, Malassezia furfur, Pityropsporon orbiculareorovale, Cryptococcus neoformans, Aspergillus fumigatus, Aspergillus nidulans, Aspergillus niger* and other *Aspergillus spp.,* Zygomycetes (e.g., *Rhizopus* and *Mucor), Paracoccidioides brasiliensis, Blastomyces dermatitides, Histoplasma capsulatum, Coccidioides immitis,* and *Sporothrix schenckii.*

The engineered peptide of the invention may be useful for inhibiting fungal organisms to treat or prevent infections such as: Aspergillosis, Blastomycosis, Candidiasis, Coccidioidomycosis, Cryptococcosis, Histoplasmosis, Paracoccidiomycosis, Sporotrichosis, Zygomycosis. In certain embodiments of the invention, the engineered peptide of the invention is effective for treating or preventing Tinea pedis, Tinea versicolor, and Onychomycosis. Other fungal infections, for which the engineered peptide of the invention is effective, include: Tinea barbae, Lobomycosis, Mycetoma, Piedra, Pityriasis versicolor, Tinea capitis, Tinea corporis, Tinea cruris, Tinea favosa, Tinea nigra, Otomycosis, Phaeohyphomycosis, and Rhinosporidiosis.

By "subject" is meant an animal, preferably a mammal such as a feline, a canine or a primate. According to a preferred embodiment, the subject to be treated is a human.

By "an effective amount" is meant an amount of pharmaceutical composition comprising at least one engineered peptide which is sufficient to induce the regression or the disappearance of symptoms related to a condition caused by a bacterium or a fungis. The doses used for the administration can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

Antimicrobial activity of the engineered peptide of the invention may be assessed by different technics well known by the skilled artisan. For example determination of minimal inhibitory concentration (MIC) uses two fold serial dilutions in microbial liquid media. Antimicrobial activity of the engineered peptide of the invention can also be evaluated by determination of the minimal bactericidal/fungicidal concentration (MBC), i.e. the minimal concentration of the engineered peptide of the invention killing 99.9% of the bacteria or fungi.

The therapeutic index (TI) can also be useful to assess the antimicrobial safety of the engineered peptide of the invention. TI of an engineered peptide of the invention is calculated as the ratio between the minimum toxic concentration to the minimum effective concentration.

Biofilms are responsible for approximately 60% of nosocomial infections. They are essentially due to microbial colonization of implanted biomaterials. Eradication of a bacterial biofilm is a major clinical problem considering that antibiotics normally active on bacteria in planktonic state often turn out to be much less effective against structures organized into a biofilm.

In a particular embodiment, the peptide of the invention is used to treat a condition caused by a bacterium involving biofilm formation such as cystic fibrosis, endocarditis, cystitis, infections caused by indwelling medical devices, dental plaque formation or periodontitis.

The use of antibiotics has changed the natural evolution of bacteria by reducing susceptible pathogenic populations and increasing resistant populations. Resistance is often associated with a fitness cost because the genetic burden required for resistance may be deleterious in antibiotic-free environments. In this case, restriction of the use of antibiotics has been proposed, with the aim of eradicating resistant bacteria. However, the genetic background of resistant pathogens allows them to persist in the presence of minimal concentrations of antibiotics or even in the absence of these. Hypermutation, compensatory mutations, and cross co-selection are a few of the many mechanisms that favor the persistence of resistant pathogens and even, in some cases, selection of the most virulent and most resistant pathogens. The engineered peptide of the invention may be an interesting alternative to the use of antibiotics.

In some embodiments, the engineered peptide of the invention can used in conjunction with a further (e.g., a second) anticancer therapy. For example, the anticancer therapy can be a surgical therapy, chemotherapy, radiation therapy, gene therapy or immunotherapy. In some aspects, if the anticancer therapy is a chemotherapy, in may be preferred that the chemotherapy comprise one or more apoptosis inducing agents.

In a particular embodiment, the present invention relates to the use of an engineered peptide as defined above as anti-proliferative agent.

In another aspect, the present invention relates to the *in vitro* use of an engineered peptide as defined above as disinfectant or preservative agent.

By "disinfectant" is meant substances, agents, products suitable for the killing of microorganisms, and in particular microorganisms or fungus.

By "preservative agent" is meant chemical added to a food or material to prevent the growth of microorganisms, and in particular microorganisms or fungus.

The food preservation is still an important issue, as improper food preservation results in the health problem. Among bacteria and fungus responsible for food contamination, *Salmonella spp., Shigella spp., Micrococcus spp., Enterococcus faecalis, Bacillus licheniformis, Escherichia coli, Listeria monocytogenes, Staphylococcus aureus, Campylobacter jejuni, Yersinia enterocolitica, Vibrio parahemolyticus, Escherichia coli 0157:H7, Clostridium botulinum, Saccharomyces cerevisiae* and *Penicillium expansum* are the most frequent microbial agents found in dairy products, meat or fish products and vegetable and juices.

In a particular embodiment, the in vitro use of an engineered peptide according to the invention is to enhance the storage life of food products by preventing the growth of food-spoilage organisms.

In still another aspect, the present invention relates to a medical device or implant comprising a body having at least one surface coated with or including an engineered peptide as defined above.

By "medical device or implant" is meant devices that are placed inside or on the surface of the body. Many implants are prosthetics, intended to replace missing body parts. Other implants deliver medication, monitor body functions, or provide support to organs and tissues. Medical device or implant are made from metal, plastic, ceramic or other materials and can be placed permanently or they can be removed once they are no longer needed. For example, stents or hip implants are intended to be permanent. The device or implant may be, for example, intravascular, peritoneal, pleural and urological catheters; heart valves; cardiac pacemakers; vascular shunts; coronary stunts; dental implants or orthopedic or intraocular prosthesis.

This type of medical device or implant and the uses and methods of preparation thereof are described for example in patent application WO 2005/006938.

The surface coated with or including an engineered peptide according to the invention may be composed of thermoplastic or polymeric materials such as polyethylene, Dacron, nylon, polyesters, polytetrafluoroethylene, polyurethane, latex, silicone elastomers and the like, or of metallic materials such as gold. In a particular embodiment, the peptide of the invention is covalently attached to a functionalized surface, preferably a metallic surface, via its N-terminal or C-terminal end. Optionally, the peptide may be attached to the surface through a spacer arm.

Preferably, the surface may be coated with an engineered peptide at a density of about 1 $\mu$g/cm$^2$ to about 50 mg/cm$^2$, preferably from about 100 $\mu$g/cm$^2$ to about 5 mg/cm$^2$.

The following examples are provided to illustrate certain preferred embodiments and aspects of the present invention and are not to be construed as limiting the scope thereof.

## EXAMPLES

## MATERIAL AND METHODS

### Venom preparation

Venoms were supplied by Latoxan (Portes-Lès-Valence, France). The initial stocks of venoms were prepared in HPLC plus water at a concentration of 10 mg/mL. Each solution was clarified by centrifugation (5 minutes at 5000 rpm). The supernatant was stored at -80°C until use. The process was the same for venom fractions and sub fractions.

### Chromatographic identification and purification of antimicrobial peptides from *Polistes gallicus* venom

Solvents were purchased from Sigma Aldrich (Saint-Louis, MO, USA). To identify antimicrobial active peptides, lyophilized *Polistes gallicus* venom (50 mg) was dissolved in HPLC water and then centrifuged at 6000 x g for 5 min at 4 °C. The supernatant was then filtered through 0.45μm nylon membrane and was used to purify the peptides. The filtered venom was fractionated by HPLC (Agilent 1100, column VaripPrep 250/21, 100-5 μm RP-C18 Machery nagel NUCLEODUR under a linear gradient from 0 to 80% (v/v) $CH_3CN$ (containing 0.1% (v/v trifluoroacetic acid TFA) at a flow rate of 6 ml/min over 80 min and monitored at UV 214 nm and 280 nm. The twenty five HPLC fractions (P2 to P11 and G2 to G15) were manually collected every 3min and dried. The seven fractions presenting inhibitory activity (P2, P3 and G7 to G11) against microorganisms were re-fractionated using RP-C18 column (250/4.6; 5μm; NUCLEODUR) with isocratic elution with 50% (v/v) $CH_3CN$ (containing 0.1% v/v trifluoroacetic acid (TFA)) at a flow rate of 1ml/min. The elution was monitored at 214 nm and 280 nm and the sub-fractions were manually collected in 5 ml glass vials and then dried. The subfractions were re-submitted to bacteria growth inhibition assays. The homogeneity of each active peak was then determined by LC-MS analysis.

## Peptides characterization by mass spectrometry

### MALDI-TOF:

The molecular mass of purified natural peptides was determined using MALDI-TOF mass spectrometry. Experiments were performed on a Microflex II instrument (Bruker Daltonics, Billerica, MA, USA) operating in reflector mode in a range from 200 to 5000 Da and positive polarity. A 1 μL volume of pure peptide was mixed with an equal volume (1:1, vol:vol) of a matrix solution of a-cyano-4-hydroxycinnamic acid (HCCA) in 70% acetonitrile containing 0.1% TFA, on a stainless steel target; the droplet was allowed to evaporate before introducing the target into the mass spectrometer. Calibration was performed using the Peptide Calibration Standard.

### LC-MS:

Mass spectrometric analysis of synthetic peptides were performed on LC/MS (LC-2010A HT Liquid Chromatograph Shimadzu, Marne la Vallée, France) using an ESI source operated under negative mode. LC/MS separation was achieved on a C18 NUCLEODUR 250 mmx4.6 mm at 35 °C. The detection wave lengths were 214 and 280 nm. Elution was carried out with solvents A and B containing acid formic. A linear gradient from 0 to 80% solvent B for 80 mn was used. The flow rate was set up at 1 ml/mn. The mass of peptide has been calculated according to the following formula: m/z= (Mproduct + (z * Madduct) )/z. Adduct products are: m/z + $H^+$ (1 g/mol), or m/z + $Na^+$ (23 g/mol), or m/z + $K^+$ (39.1 g/mol).

## Amino acid analysis

In order to confirm the peptide primary structure and to quantify all used samples, an amino acid analysis was made after acid hydrolysis [6N HCl, 72 h, 110 °C], at Institut de Biologie Structurale (Grenoble, France).

## N-terminal sequencing, molecular mass determination and peptide mass fingerprinting

N-terminal sequence analysis was performed using Procise® LC 494 Protein Sequencing System (Applied Biosystems, Villebon sur Yvette, France). Peptides were diluted into 100μL of water/0.1% TFA and applied onto a (Prosorb) methanol treated PVDF membrane of Prosorb. The membrane was then washed with 100μL of water/0.1% TFA, cut out, dried and introduced into the protein sequencer. A number of N-terminal sequencing cycles, corresponding to the number of expected amino acids, was performed. Briefly, peptide's amino-terminal amino acid was specifically reacted with phenylisothiocyanate (PITC) in basic conditions (N-methylpiperidine) at 45°C. In acid conditions (TFA), this derivatized amino acid was then selectively removed, leaving the rest of the peptide chain intact. Each cycle of the degradation removed the new amino-terminal amino acid from the peptide chain. The resulting PhenylThioHydantoin-amino acid (PTH-AA) was analyzed sequentially to determine the amino acid sequence of the peptide. Analysis of resulting PTH-AA was performed by RP-HPLC on an Applied PTH C18 (2,1mm x 22cm, 5μm) thermostated at 55°C. Elution was monitored at 269 nm and realized following manufacturer's protocol (0,325mL/min - gradient of ACN/isopropanol (buffer B) in water + 3,5% tetrahydrofuran adjusted at pH4 with sodium acetate). A PTH-amino acid standard containing the PTH derivative of the 20 commonly occurring amino acids is required for peak identification and calibration of sequencer performance.

## Chemical synthesis of gallicune peptides and physicochemical characterization of the synthetic products

The peptides were obtained by the solid-phase method using an automated peptide synthesizer (Model 433A, Applied Biosystems Inc.). Peptide chain was assembled stepwise on Rink amide resin (0.69 mEq of amino group/g) using 1 mmol of N-9-Fluorenylmethyloxycarbonyl (Fmoc) amino acid derivatives. The side chain-protecting groups were as follow: trityl for Cys and Asn; tert-butyl for Ser, Thr, Glu, and Asp; pentamethylchroman for Arg, and tert-butyloxycarbonyl for Lys and Trp. N-alpha-Amino groups were deprotected by treatment with 18% and 20% (v/v) piperidine/N-methylpyrrolidone for 3 and 8 min, respectively. The Fmoc-amino acid derivatives were coupled (20 min) as their hydroxybenzotriazole active esters in N-methylpyrrolidone (four-fold excess). After peptide chain assembly, the linear peptides and the peptide resin containing Cys amino acid residue were treated for 3 h at room temperature with a mixture of TFA/Triisopropylsilane /water (90:5:50, v/v) and TFA/H2O/thioanisole/Triisopropylsilan (88:5:5:2, v/v) in the presence of crystalline phenol (2.25 g), respectively. The peptide mixture was then filtered, and the filtrate was precipitated by adding cold t-butylmethyl ether. The crude peptide was pelleted by centrifugation (5000Ug; 10 min) and the supernatant was discarded. The reduced peptide containing Cys amino acid residue peptide was then dissolved in 200 mM Tris HCl buffer, pH 8.3, at a final concentration of 2.5 mM and stirred under air to allow oxidation/folding (72 h, room temperature). The target product was feeze-dried and purified to homogeneity, by reversed-phase high pressure liquid chromatography (RP-HPLC) on semi-preparative column by means of a 100-min linear gradient of 0.08% (v/v) trifluoroacetic acid (TFA)/0 to 100% acetonitrile in 0.1% (v/v) TFA/H$_2$O at a flow by: (i) analytical C18 reversed-phase HPLC (Machery Nagel, C18 5 $\mu$m, 4.6 x 250 mm) using a 100-min linear gradient of 0.08% (v/v) TFA/0 to 100% acetonitrile in 0.1% (v/v) TFA/H$_2$O at a flow rate of 1 ml/min; (ii) amino acid analysis after acidolysis (6 N HCl/2% (w/v) phenol, 20 h, 118°C, N2 atmosphere); and (iii) mass determination by matrix-assisted laser desorption ionization to time of flight mass spectrometry (Merrifield B. Science 1986 Apr18;232(4748):341-7; Mabrouk et al., Biochim Biophys Acta. 2007 Oct;1768(10):2528-40).

## Antimicrobial tests (MIC testing)

### Bacterial Strains and Media

Bacterial strains used in this study were reference strains and were obtained from either the American Type Culture Collection (ATCC), the German Leibniz Institute (DSMZ) or the French Pasteur Institute (CIP). Gram negative bacterial strains used were: *Acinetobacter baumannii* multi resistant strain (MRS) (CIP 110431), *Citrobacter farmeri* (ATCC 51631), *Citrobacter rodentium* (ATCC 51116), *E. coli* (ATCC 8739), enterohemorrhagic *E. coli* -EHEC (K88), *Helicobacter pylori* (ATCC 43504), *Klebsiella pneumoniae* (DSM 26371), *Pseudomonas aeruginosa* (ATCC 9037), fluroroquinolone- and carbapenem-Resistant *Pseudomonas aeruginosa* (CIP 107398), *Pseudomonas aeruginosa* 01, *Salmonella enterica* (CIP 80.39), *Shigella flexneri* (ATCC 12022), *Vibrio alginolyticus* (DSM 2171). Gram positive bacterial strains used were: *Arthrobacter gandavensis* (DSM 2446), *Bacillus cereus* (Bacillus subtilis (ATCC 6633), Nisin-resistant B. *subtilis* (DSM 347), *Lactococcus lactis* (MG1363), *Staphylococcus aureus* (ATCC 6538P), methicillin resistant *S. aureus* strain MRSA USA300 (ATCC BAA-1717 USA 300 CA-MRSA) and *Streptococcus pyogenes* (DSM 20565). In addition to Gram negative and Gram positive strains, *Mycobacterium smegmatis* (mc2155, ATCC 700084) was also used as model of Mycobacterium species. Bacteria were cultured as previously described. Briefly, all bacterial strains (except *H. pylori* and *M. smegmatis* were grown on Luria Bertani (LB) agar plates and in LB broth at 37 °C in aerobic condition. *M. smegmatis* was cultured in Middlebrook 7H10 agar plate and Middlebrook 7H10 broth at 37 °C in aerobic condition. *M. smegmatis* was cultured in Brain Heart Infusion (BHI) agar plates and BHI broth supplemented or not with fetal calf serum (10%) at 37 °C in an anaerobic cabinet (Coy Laboratory Products, Grass Lake, MI). *H. pylori* was grown in BHI in micro-aerobic condition using micro-aerobic BD GasPack generator. *Candida albicans* was grown on LB agar plates and in liquid RMPI medium (buffered with MOPS of final concentration 0.165 M for pH 7.0 from Thermofisher).

### Evaluation of Antimicrobial activity

Antimicrobial activities were determined as previously described (Di Pasquale et al., Chem Phys Lipids. 2010 63(2):131-40; Olleik et al., European Journal of Medicinal Chemistry 2019 Mar 1;165:133-141) by determination of their minimal inhibitory concentration (MIC) using two-fold serial dilutions in bacterial liquid media following the National Committee of Clinical Laboratory Standards (NCCLS, 1997). Briefly, for most of the bacteria, single colonies of the different bacterial strains cultured on specific agar plates were used to inoculate 3 mL of Mueller-Hinton (MH), *H. pylori* and *M. smegmatis* that were cultured in 3 mL of BHI or Middlebrook 7H10 broth, respectively. Tubes were then incubated overnight (for approximately 16 h) at 37 °C under stirring (200 rpm), except for *M. smegmatis* that was left to grow for 48-72 h. Optical density (OD) of the bacterial suspensions were then read at 600 nm, adjusted to 1 with medium before bacteria were diluted 1/100 in 3 ml of fresh medium and incubated at 37°C, 200 rpm until bacteria reached log phase growth (OD 600nm around 0.6). In the case of *H. pylori* and *M. smegmatis,* MIC were performed directly using late phase growing suspension without preparing log phase growth suspension. Bacteria were diluted in medium to reach bacterial density around $10^{4-5}$ bacteria/ml. 100 $\mu$L per well of bacterial suspension were then added into sterile polypropylene 96 well microplates (Greiner BioOne) already containing 100 $\mu$l of medium with increasing concentrations of peptides obtained by serial dilution (from 0 to 100 $\mu$M, 1:2 dilution). Plates were incubated at 37 °C for 18-24 h for all bacteria except *M.*

*smegmatis* that where incubated for 72 hours. All bacterial strains were tested in aerobic conditions. MIC on *H. pylori* was performed in a micro-aerobic atmosphere generated using anaerobic BD GasPack system. For *C. albicans,* single colonies obtained from PD agar plate were collected and resuspended in Roswell Park Memorial Institute (RMPI) medium (buffered with MOPS of final concentration 0.165 M for pH 7.0 from Thermofisher at $10^3$ *C. albicans* cells/ml before being added to the 96-wells plates in the presence of increasing concentrations of test compounds. Plates were then incubated at 35 °C during 24 hours before reading. At the end of the incubation, OD600nm was measured using microplate reader (Synergy Mx, Biotek). The MIC was defined as the lowest concentration of drug that inhibited visible growth of the organism. Experiments were conducted in independent triplicate (n = 3). Antimicrobial activity of peptides was also evaluated by determination of the minimal bactericidal /fungicidal concentration (MBC), i.e. the minimum concentration of the test compound killing 99.9% of the bacteria or fungi. MBCs were measured by streaking 10 μL of wells with no observed growth of previously prepared MIC plates on agar plates. After incubation in the proper condition, numbers of colonies were counted. Concentrations where ≤ 1 colony grew were considered the MBC.

## Selection for resistant bacteria

To evaluate if populations of AMP-resistant bacteria could be selected, cultures were continuously exposed to LTX-gallicune-1 (I1N1) and LTX-gallicune-2(R1W8) peptides for a duration of 30 days, as previously described (Oyama et al., 2017 Biofilms Microbiomes, Dec 1;3:33). Briefly, broth microdilution susceptibility testing was performed using a standard doubling-dilution series of gallicune peptides concentrations on day 1. Following incubation of the cultures for 24 h, and determination of the MIC, the well that contained the highest concentration of AMPs permitting growth was diluted 1: 1000 in MH and used to provide the inoculum for the next MIC assay; this process was repeated daily for 30 days.

## Hemolytic activity assay

The hemolytic activity of each peptide was determined by the leakage of hemoglobin from human erythrocytes (obtained from Divbioscience, NL) as previously described (Oyama et al., 2017 Biofilms Microbiomes, Dec 1;3:33).
Briefly, human erythrocytes were washed 3 times with phosphate buffer saline (PBS) and concentrated using centrifugation at 800 g for 5 min. Human erythrocytes were then resuspended in PBS at a final concentration of 8 %. 100 μl of human erythrocytes were then added per well of sterile 96 well microplate already containing 15 μl of serially diluted antimicrobials (1:2 in distilled water). After 1 h at 37°C, the microplate was centrifuged at 800 g for 5 min. 100 μl were then carefully collected and transferred to a new 96 well microplate and OD was measured at 405 nm. Hemolysis caused by peptides was expressed as percentage of hemolysis; Triton-X100 at 0.1 % (v:v) was used as positive control and gave 100 % hemolysis. Hemolysis rate was determined as below:

$$\text{Hemolysis rate (\%)} = (A - A2)/(A1 - A2) \times 100\%$$

where A is the absorbance of indicator mixtures with different factors, A1 and A2 are the positive control, and the negative control, respectively.

## Cytotoxicity assay

Normal human cells used were BEAS-2B (ATCC CRL-9609), IMR-90 (ATCC CCL-186) and Human Umbilical Vein Endothelial Cells (HUVEC) (from Sigma-Aldrich) corresponding respectively to human normal lung epithelial, normal lung fibroblast cells and human normal endothelial cells. All cells except HUVEC cells were cultured in Dulbecco's modified essential medium (DMEM) supplemented with 10% fetal calf serum (FCS), 1% L-glutamine and 1% antibiotics (all from Invitrogen). HUVEC cells were cultured in specific endothelial media (from Sigma Aldrich). Cells were seeded onto 25 cm flasks maintained in a 5% $CO_2$ incubator at 37 °C. Cells grown on 25 $cm^2$ flasks were detached using trypsin-EDTA solution (from Thermofisher), counted using Mallassez counting chamber and seeded into 96-well cell culture plates (Greiner bio-one) at approximately 10,000 cells per well. The cells were left to grow for 48-72 h at 37 °C in a 5% $CO_2$ incubator until they reached confluence. Plates were then aspirated and increasing concentrations of peptides diluted in culture media were added to the cells for 48 h at 37 °C in a 5% $CO_2$ incubator. At the end of the incubation, wells were emptied and cell viability was evaluated using Resazurin based in vitro toxicity assay kit (from Sigma-Aldrich) following manufacturer's instructions. Briefly, Resazurin stock solution was diluted 1:10 in sterile PBS containing calcium and magnesium (PBS, pH 7.4) and empty wells were filled with 100 μL of the diluted solution. After 4 h incubation at 37 °C, fluorescence intensity was measured using microplate reader (Synergy Mx, Biotek) with an excitation wavelength of 530 nm and an emission wavelength of 590 nm. The fluorescence values were normalized by the controls (buffer treated cells) and expressed as percent viability. The IC50 values of peptides on cell viability (i.e. the concentration of derivative causing a reduction of 50% of cell viability) were determined using GraphPad® Prism 7 software. t-Test and two way ANOVA analyses were used to address the significant differences between mean values with significance set at $p < 0.05$.

## Permeabilization of the cytoplasmic membrane

The effects of synthetic antimicrobial peptides on the cell membrane integrity of bacteria were investigated using propidium iodide (PI), a viable cell-resistant DNA / RNA marker as previously described (Di Pasquale et al., Chem Phys Lipids. 2010 63(2):131-40; Oyama et al., 2017 Biofilms Microbiomes, Dec 1;3:33).

Logarithmic growing bacterial suspension was prepared by diluting over-night bacterial suspension of bacteria in fresh liquid broth (1 in 10 dilution). After 3 h incubation at 37°C, 200 rpm, bacteria were pelleted by centrifugation for 5 min at 3000 g. Bacterial cell pellet was then resuspended in sterile PBS at about 109 bacteria / ml. Propidium iodide (solution at 1 mg/ml from Sigma Aldrich) was added to the bacterial suspension at a final concentration of 60 $\mu$M. 100 $\mu$l of this suspension were then transferred into 96-well black plates (Greiner Bio-one) already containing 100 $\mu$l of serially diluted peptides (1:2 in PBS). Kinetics of fluorescence variations (excitation at 530nm / emission at 590 nm) were then recorded over time at 37°C using a microplate reader (Synergy Mx, Biotek), Cetyl trimethylammonium bromide (CTAB) (at 300 $\mu$M) being used as positive control giving 100 % permeabilization, Peptide's effect being expressed as percentage of total permeabilization.

## Evaluation of the stability of the peptides to proteases and human serum

In order to evaluate the stability of the peptides to condition they will face in vivo, their resistance to proteases and human serum was evaluated (Oyama et al., 2017 Biofilms Microbiomes, Dec 1;3:33). Briefly, one volume of peptides (at 1 mM) was diluted in one volume of proteases (Pepsin, Trypsin, Chymotrypsin, Papain all from Sigma Aldrich and diluted at 2 mg/ml in HCl 10 mM pH2 for Pepsin, PBS pH 7 for Trypsin and Chymotrysin and PBS pH 6 for Papain) or human serum (Sigma Aldrich H4522). Peptides incubated in the same conditions in PBS were used as controls. After 4 h (for proteases) and 4 or 24 h (for human serum) of incubation at 37°C, PMSF was added to the reaction mixture at a final concentration of 100 $\mu$M to stop the digestion. MIC of the digested peptides were then determined as explained above.

## Antitumor activity of the peptides

Human cancer cell lines (Mia PaCa-2 (CRL-1420), PC-3 (CRL-1435), MDA-MB-231 (HTB-26)) and normal lung fibrobasts (BEAS-2B (ATCC CRL-9609) and IMR-90 (ATCC CCL-186)) were obtained from American Type Culture Collection (ATCC). HUVEC cells were obtained from Sigma Aldrich and were grown in defined all-in-one ready to use endothelial cell growth medium (Sigma Aldrich). Other cells were cultured in Dulbecco's modified Eagle's medium (DMEM, Gibco Life Technologies) supplemented with 10% heat-inactivated fetal calf serum (FCS, Lonza) at 37 °C in an incubator containing 5% CO2. Cells were sub-cultured in new media every 3-4 days. Antiproliferative / antitumor effects of the peptides were measured as previously described (Borie et al., Eur J Med Chem. 2018;148:306-313). Briefly, cells were detached from growing flask using trypsin, cells were counted and seeded in 96 well-plates in the growth media at an initial cell density of 5000 cells/well. Cells were left for 4 h to attach to the wells. Cells were then treated with increasing concentrations of peptides diluted in culture media. After 48 h incubation at 37 °C in a 5% CO2 incubator, wells were emptied and cell number was evaluated using Resazurin based in vitro toxicity assay kit (from Sigma-Aldrich) following manufacturer's instructions. Briefly, Resazurin stock solution was diluted 1:10 in sterile PBS containing calcium and magnesium (PBS, pH 7.4) and empty wells were filled with 100 $\mu$L of the diluted solution. After 4 h incubation at 37 °C, fluorescence intensity was measured using microplate reader (Synergy Mx, Biotek) with an excitation wavelength of 530 nm and an emission wavelength of 590 nm. The fluorescence values were normalized by the controls (buffer treated cells) and expressed as per-cent viability. The IC50 values of peptides on cell division / proliferation (i.e. the concentration of derivative causing a reduction of 50% of cell division) were determined using GraphPad® Prism 7 software. t-Test and two way ANOVA analyses were used to address the significant differences between mean values with significance set at $p < 0.05$.

## Anti-inflammatory activity of the peptides

Anti-inflammatory activity of the peptides was determined using eLUCidate™ HeLa TLR4/IL-8 Renilla Luciferase Reporter cell line (obtained from ASMBIO). eLUCidate™ HeLa cells were cultivated according to manufacturer instructions. eLUCidate™ HeLa cells were seeded in 96 wells plates at an initial 50,000 cells/well. The next day, media from wells was empty and cells were exposed to LPS from P. aeruginosa (from Sigma Aldrich) diluted in culture media at a final concentration of 10 ng/ml in the presence of increasing concentrations of peptides. Polymyxin B (PMB, from Sigma Aldrich) was used as positive control. After 6 h incubation at 37°C in an incubator containing 5% CO2, wells were empty and cells were lysed in 70 μl of PBS containing 1% Triton X100 for 15 min on ice. 50 μl of cell lysats were then transferred to white 96 wells plates containing 50 μl of Renilla Luciferase substrate (Genofax A from Yelen, Marseille France). Luminescence intensities were measured using microplate reader (Synergy Mx, Biotek). The luminescence values were normalized by the controls (buffer treated cells) and expressed as percent of LPS stimulation. The IC50 values of peptides on LPS effect (i.e. the concentration of derivative causing a reduction of 50% of LPS effect) were determined using GraphPad® Prism 7 software. t-Test and two way ANOVA analyses were used to address the significant differences between mean values with significance set at $p < 0.05$.

## RESULTS

### Peptide purification:

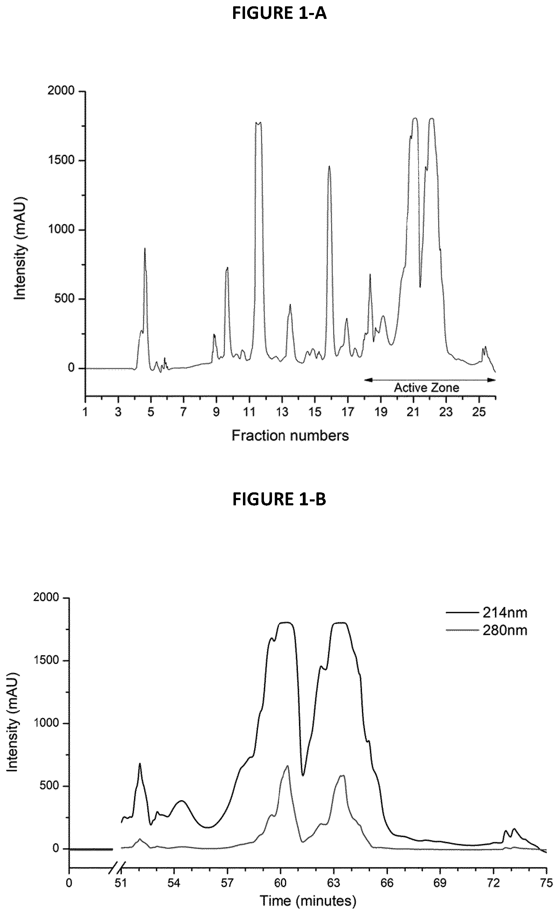

Peptides from crude venom were separated by RP-HPLC. As illustrated in Fig.1A the venom was fractionated into 25 fractions. Antimicrobial activity was tested for each fraction and the one eluting at 60 min (marked by an arrow) showed the strongest effect. This fraction, named gallicune, was further re-purified using reverse-phase HPLC (Fig 1B). Three well-resolved peaks were obtained eluting between 49% and 55% of acetonitrile. Peptides with antimicrobial activity were found in the marked fraction eluted at a retention time of approximately 60 min at 60% ACN (Fig 1B).

### Sequence of peptides:

MALDI-TOF mass analysis indicated the molecular mass of gallicune peptides were 1855.37 Da, 1910.34 Da, 1266.87 Da.

The amino acid sequences of galicune peptides were determined by combination of automated Edman degradation, amino acid analysis and MS/MS.

An online BLAST search showed that peptide sequences with molecular weights of 1855.37 Da and 1910.34 Da correspond to Dominulin A and Dominulin B sequences, isolated from *Polistes dominulis,* respectively.

The peptide having a molecular weight of 1266.87 Da, composed of 12 amino acids (Ile1-Leu 12) and called Gallicus Chemotactic Peptide (Gallicus-CP) I1L12, belongs to the family of chemotactic peptides. Its sequence ILSAILGLLKNL is disclosed in table 1.

### Structure-activity Relationship Study using gallicus-CP (I1L12) and LTX-gallicune-1-(I1N11) analogs:

### A- Peptide Engineering:

In order to obtain the Gallicus-CP (I1L12) peptide in a non-limiting quantity, it was synthesized chemically by the solid phase method. Obtaining several analogs deleted at the N- or C-terminal ends makes it possible to determine the shortest active sequence. The use of non-natural or exotic amino acids, modifications of the N- and C-termini as well as cyclization with or without a disulfide bridge make it possible to obtain more stable analogs/derivatives to enzymatic degradations. Thus all of the Gallicus-CP analog peptides appearing in the table were synthesized purified and characterized by analytical HPLC for their homogeneity and by mass spectrometry to confirm their molecular weight.

Our objective was to design peptide analogs/derivatives, more active than Gallicus-CP against gram⁻ and/or gram⁺ bacteria, less toxic, shorter, stable, and pore-forming.

The design of 2 new and improved AMP Gallicus-CP I1L12 derivatives has been completed:

- the LTX-gallicune-1 sequence was obtained by replacing the residues Ser in position 3, Leu in position 6 and Gly in position 7 with three lysines, while keeping the isoleucine 1 and lysine 10 residues essential for antibacterial activity (Table 2). Thus, the sequence of LTX-gallicune-1(I1-N11) is ILKAIKKLLKN. By deleting the asparagine in C-ter of LTX-gallicune-1(I1-N11) peptide, the inventor obtained the LTX-gallicune-1(I1-K10) peptide of sequence ILKAIKKLLK. The improvement of the stability of the LTX-gallicune-1(I1-N11) peptide in human serum and proteases was achieved by synthesizing peptides composed of D- amino acids resistant to proteases and peptidases (Table 2).

- the peptide LTX-gallicune-2 (R1W8) was generated from the highly active inverso sequence by substituting the residues in position 8, 9 and 10 (isoleucine, alanine, serine) with tryptophan in position 8 and 10, and a leucine in position 9 and a leucine in Gallicus-CP I1L12. Tryptophan has a substantial affinity for the interface between the lipid bilayer and the aqueous medium and helps to anchor peptides to the surface of the lipid bilayer. The sequence of LTX-gallicune-2 (R1W8) is RLLGLWLW.

**B- Antimicrobial activities:**

Antimicrobial activity of synthetic Gallicus-CP and its analogs against Gram-positive, Gram-negative bacteria and fungi was tested. Amoxicilline, Gentamycine and mellitine were included as control for antibacterial potential. Results are presented hereafter in table 3 and table 4.

**Table 3: MIC of engineered peptides derived from Gallicus-CP(11L12) against *P. aeruginosa, S. aureus, E. coli* et *B. subtilis* growth**

| Peptides | MIC ($\mu$M) | | | |
|---|---|---|---|---|
| | *P. aeruginosa* | *S. aureus* | *E. coli* | *B. subtilis* |
| Gallicus-CP($I_1L_{12}$) | 100 | 6.25-3.1 | 100-50 | 3.13-1.56 |
| $I_1L_9$ | / | 100 | / | 100 |
| $I_1K_{10}$ | / | 50-25 | 100-50 | 25-12.5 |
| $K_{10}I_1$ | / | 12.5-6.25 | 100-50 | 6.25-3.13 |
| $L_2K_{10}$ | / | / | / | 100 |
| $L_{12}I_1$ | 50 | 3.13-1.56 | 50-25 | 3.13-1.56 |
| $(i_1I_{12})$ | 100 | 6.25 | 100 | 3.13 |
| $K_1I_8$ | / | 25 | 50 | 12.5-6.25 |
| LTX-gallicune-2 ($R_1W_8$) | / | 12.5 | / | 12.5 |
| LTX-gallicune-1 ($I_1N_{11}$) | 6.25-3.12 | / | 6.25 | 12.5-6.25 |
| **Antibiotics** | | | | |
| Melittine | 6.25 | 1.56 | 6.25-12.5 | 1.56 |
| Amoxicilline | / | 0.9 | 9 | 0.45-0.9 |
| Gentamycine | 0.3 | NT | NT | NT |

« / » means MIC > 100$\mu$M. NT = Not Tested. For all conditions, n=4 unless specified. The upper case letter indicates a normal L-type amino acid and the lower case letter indicates a D-type amino acid.

Moreover, the activity of the engineered peptides of the invention was compared to that of melittin and antibiotics of reference: amoxicillin and gentamycin. Melittin is an antimicrobial peptide of 26 amino acid residues known for its broad spectrum of antibacterial activity, with improved efficacy against Gram+ strains. This was confirmed on our strains with activity against gram-positive and negative, pathogenic and non-pathogenic bacteria ranging from 1.56 $\mu$M against *S. aureus* and *B. subtilis* to 12.5 $\mu$M against E. coli. The activity of the LTX-gallicune-1-(I1N11) peptide is closest to that of melittin.

It should be noted that the presence of the more hydrophobic tryptophan in place of isoleucine at the C-terminus and the substitution of lysine by the more polar arginine, maintains the antimicrobial activity of LTX-gallicune-2(R1W8) mainly against Gram+ strains.

**Table 4: MIC of engineered peptides derived from LTX-gallicune-1 (I1N11) against P. *aeruginosa, S. aureus, E. coli* et *A. baumani* growth**

| Peptides | MIC ($\mu$M) | | | |
|---|---|---|---|---|
| | *P. aeruginosa* | *S. aureus* | *E. coli* | *A.baumani* |
| LTX-gallicune-1 ($I_1N_{11}$) | 3.12 | / | 6.25 | 100 |
| $I_1L_9$* | 6.25 | NT | 100 | 100 |
| $I_1K_7$* | 100 | / | 100 | 100 |
| $L_2L_9$* | 25 | NT | 100 | / |
| $K_3N_{11}$* | / | NT | / | / |
| Ac $I_1N_{10}$* | 50 | NT | 100 | >100 |
| $I_1K_{10}$* | 1.5 | NT | 50 | 100 |
| $i_1k_{10}$* | 1.5 | NT | 50 | 100 |
| LTX- gallicune-1 (i1D-n11D) | 1.5 | / | / | 12.5 |

(continued)

| Peptides | MIC ($\mu$M) | | | |
|---|---|---|---|---|
| | *P. aeruginosa* | *S. aureus* | *E. coli* | *A.baumani* |
| **LTX-D(gallicune-1)** | 1.5 | / | 6.25 | 12.5-6.25 |

« / » means MIC > 100$\mu$M. NT = Not Tested. For all conditions, n=4 unless specified. The upper case letter indicates a normal L-type amino acid and the lower case letter indicates a D-type amino acid.

The LTX-gallicune-1(I1-N11) strongly inhibits the growth of Gram⁻ strains (MIC = 3.12 and 6.25 $\mu$M against *P. aeruginosa* and *E. coli* respectively). Although it also acts effectively against the growth of *B. subtilis* (MIC = 6.25$\mu$M), it is the only analogue with Gram⁺ / Gram⁻ activity which has a low activity against *S. aureus* (MIC $\geq$ 100$\mu$M). LTX-gallicune-1(I1-N11) peptide is rather an anti-Gram negative, unlike the LTX-gallicune-2 (R1-W8) peptide which seems to be Gram⁺ specific. LTX-gallicune-1(I1-N11) also inhibits the growth of *Candida albicans* (MIC = 50 $\mu$M).

The LTX-gallicune-1 I1-N11 and I1K10 peptides and their derivatives are the most active engineered peptides. These peptides and their functional derivatives were tested on different bacteria. Their minimal inhibition concentration (MIC) and minimal bactericide concentration (MBC) are summarized in tables 5 and 6 below.

**Table 5: MIC of engineered peptides derived from LTX-gallicune-1 (I1N11), 11K10 and their functional derivatives against bacteria**

| MIC ($\mu$M) | **I1N11** | **I1(d)N11(d)** | **(I1N11)d** | **I1K10** | **I1(d)K10(d)** | **(I1K10)d** |
|---|---|---|---|---|---|---|
| *L. lactis* | 1.5 | 1.5 | 1.5 | 3.125 | 0.78 | 1.5 |
| *B. subtilis* (Nisin-resistant) | 12.5 | 6.25 | 6.25 | 12.5 | 3.12 | 12.5 |
| *A. gandavensis* | 3.12 | 1.5 | 1.5 | 3.12 | 0.78 | 1.5 |
| *E. coli EHEC K88* | 100 | 25 | 50 | 100 | 6.25-12.5 | 50 |
| *P. aeruginosa* | 6.25 | 1.5 | 1.5 | 3.125 | 0.78 | 3.125 |
| *P. aeruginosa FQR* | 25 | 6.25-12.5 | 6.25-12.5 | 12.5-25 | 3.12-6.25 | 12.5-25 |
| *P. aeruginosa 01* | 25 | 6.25 | 6.25 | 12.5 | 3.125 | 12.5 |
| *S. flexneri* | 50 | 12.5 | 12.5 | 25 | 3.12-6.25 | 12.5 |
| *C. farmeri* | 12.5 | 6.25 | 6.25 | 25 | 3.12 | 12.5 |
| *C. rodentium* | 50 | 25 | 12.5 | 100 | 12.5 | 50 |
| *S. enterica* | 100 | 25 | 25 | 50 | 12.5 | 50 |
| *V. alginolyticus* | 0.78 | 0.78 | 0.78 | 1.56 | 0.78 | 0.78-1.56 |
| *H. pylori* | 100 | 25 | 50 | 50 | 12.5 | 50 |
| *M. smegmatis* | 25 | 25 | 25 | 50 | 12.5 | 6.25 |

The results presented in table 5 show that LTX-gallicune-1D analogues (i1n11, i1k10, I1K10) are the most active peptides against *P. aeuroginosa* with an MIC ranging from 0.78 to 3.125 micromolar.

**Table 6: MBC of engineered peptides derived from LTX-gallicune-1 (11N11), 11K10 and their functional derivatives against bacteria**

| MBC ($\mu$M) | **I1N11** | **I1(d)N11(d)** | **(I1N11)d** | **I1K10** | **I1(d)K10(d)** | **(I1K10)d** |
|---|---|---|---|---|---|---|
| *L. lactis* | 3.12 | 0.78 | 1.5 | 0.78 | 1.56 | 3.12 |
| *B. subtilis* (Nisin-resistant) | 12.5 | 6.25 | 6.25 | 12.5 | 3.12 | 12.5 |
| *A. gandavensis* | 3.12 | 1.5 | 1.5 | 3.12 | 0.78 | 1.5 |
| *E. coli EHEC K88* | >100 | 50 | 50 | 100 | 6.25 | 50 |
| *P. aeruginosa* | 6.25 | 1.5 | 1.5 | 3.12 | 1.5 | 3.12 |
| *P. aeruginosa FQR* | 100 | 25 | 12.5 | 100 | 25 | 100 |
| *P. aeruginosa 01* | 50 | 12.5 | 25 | 50 | 12.5 | 50 |
| *S. flexneri* | 50 | 12.5 | 12.5 | 100 | 6.25 | 12.5 |

(continued)

| MBC (μM) | I1N11 | I1(d)N11(d) | (I1N11)d | I1K10 | I1(d)K10(d) | (I1K10)d |
|---|---|---|---|---|---|---|
| *C. farmeri* | 12.5 | 12.5 | 6.25 | 50 | 3.12 | 50 |
| *C. rodentium* | 50 | 25 | 25 | 100 | 12.5 | 50 |
| *S. enterica* | 50 | 25 | 25 | 50 | 12.5 | 25 |
| *V. alginolyticus* | 0.78 | 0.78 | 0.78 | 1.5 | 0.78 | 1.5 |
| *H. pylori* | 100 | 25 | 50 | 50 | 12.5 | 50 |

The results presented in table 5 show that LTX-gallicune-1D analogues (i1n11, i1k10, I1K10) are the most active peptides against *P. aeuroginosa* with an MBC ranging from 1.5 to 6.25 micromolar.

**Hemolytic activities:**

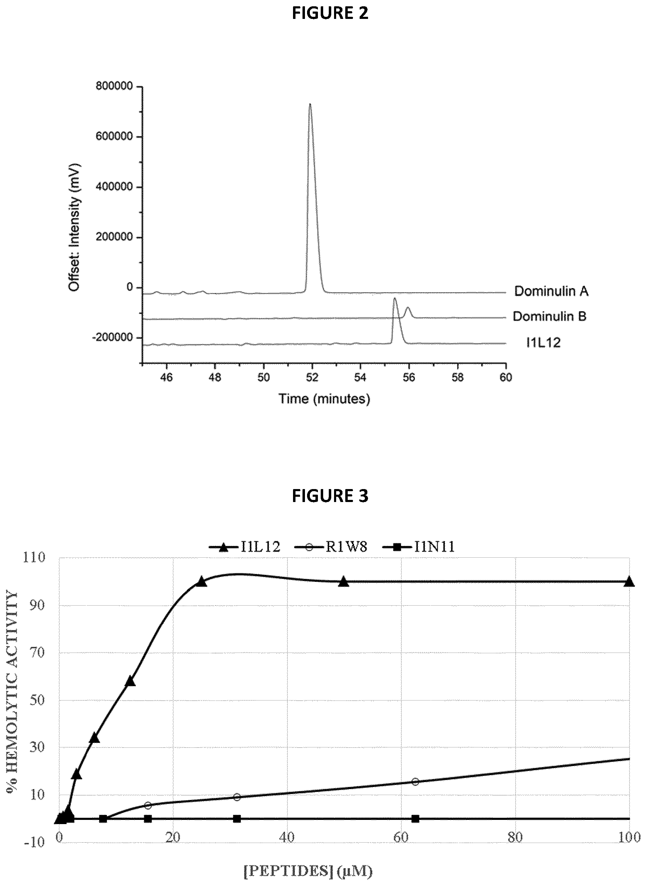

Human red blood cells were used to evaluate the hemolytic activities of the peptides. The percentage of the lysed red cells as a function of concentration of tested peptide is shown in Figure 3.

The hemotoxicity of the peptides is dose-dependent. Most peptides with high antibacterial activity also show high hemotoxicity (50% MHC <100μM) (data not shown). The toxic peptide dose for human erythrocytes is close to the dose required to inhibit bacterial growth.

The parent peptide Gallicus-CP is highly hemotoxic. Indeed, a concentration of 10 μM is sufficient to induce 50% hemolysis of human erythrocytes. In the same way, the active analogues are hemotoxic except for those whose amino acids were modified, R1W8 and especially LTX-gallicune-1-(I1N11) are respectively 20 to 1000 times less toxic (Fig 3). Since the LTX-gallicune-1-(I1N11) peptide is not hemolytic at 1mM, it has been tested up to 10 mM without exhibiting any toxicity towards red blood cells. This is also true for to LTX-gallicune-1D (i1n11, i1k10, I1K10, I1K10) and LTX-gallicune-1-I1-K10 peptides which do not exhibit any hemotoxicity up to 10 mM.

The therapeutic index of each peptide could be calculated as the ratio of MHC50 to MIC (MHC50 to MIC). The lower this index, the less difference there is between the dose likely to create a therapeutic effect and the one inducing the toxicity of red blood cells. The higher the index of a substance the less dangerous it is and the lower the risk of inducing likely side effects.

All highly active synthetic analogs with high hemolytic activity have a low therapeutic index (data not shown) with the exception of active and non-toxic peptides with a strong therapeutic index: LTX-gallicune-1 (I1-N11, I1K10), LTX-gallicune-1D analogues (i1n11, i1k10, I1K10) and LTX-gallicune-1(R1W8) (Table 7).

**Table 7: Therapeutic indices (TI) of the native Gallicus CP 11L12 and engineered AMPs LTX-gallicune-1 (11N11) and LTX-gallicune-2 (R1W8).**

| Peptide | $MHC_{50}$ | $MHC_{100}$ | Therapeutic Index ($MHC_{50}$/MIC) | | | |
|---|---|---|---|---|---|---|
| | | | *P. aeruginosa* | *S. aureus* | *E. coli* | *B. subtilis* |
| $I_1L_{12}$ | 10μM | 25μM | 0,1 | 1,6-3,2 | 0,1-0,2 | 3,2-6,4 |
| $R_1W_8$ | 225μM | 1000μM | / | 18 | / | 18 |
| I1N11 | >10000μM | >10000μM | 1600-3200 | / | 200-1600 | 800-1600 |

Both MHC (50% and 100%) of AMPs peptides against human erythrocytes as well as their corresponding therapeutic index were determined. The therapeutic index was calculated based on MHC50 values divided by the MIC determined for different species of bacteria.

LTX-gallicune 1 (I1N11) exhibits the best therapeutic index not only for gram-negative bacteria *P. aeruginosa* and *E. coli* but also for the gram-positive *B. subtilis.* LTX-gallicune 2 (R1W8) exhibits the best therapeutic index for gram-positive *S. aureus.*

**Evaluation of cell toxicity:**

Although AMPs of the invention were found none hemolytic, their toxicity on human nucleated cells has to be tested. Human cells used were normal cells: IMR90 (lung fibroblast), BEAS (lung epithelium) and HUVEC (endothelium). Results can be found in Table 8 below.

**Table 8 IC50 of engineered peptides of the invention against IMR90, HUVEC and BEAS cells and their corresponding therapeutic index (TI).**

| Cytotoxicity IC50 ($\mu$M) and MIC ($\mu$M) for *P. aeruginosa* strain | | | | | | |
|---|---|---|---|---|---|---|
| | I1N11 | I1(d)N11(d) | (I1N11)d | I1K10 | I1(d)K10(d) | (I1K10)d |
| MIC PA | 6.25 | 1.5 | 1.5 | 3.125 | 0.78 | 3.125 |
| IMR90 | 892.5 | 362.1 | 653.1 | 368.4 | 782.7 | 364.8 |
| HUVEC | 825.9 | 435.5 | 869 | 781.8 | 814.3 | 682.8 |
| BEAS | 820.9 | 390.8 | 843.4 | 389.8 | 866.6 | 725.8 |
| Therapeutic index (IC50s compared to MIC *P. aeruginosa*) | | | | | | |
| | I1N11 | I1(d)N11(d) | (I1N11)d | I1K10 | I1(d)K10(d) | (I1K10)d |
| IMR90 | 142.8 | 241.4 | 435.4 | 117.9 | 1003.5 | 116.7 |
| HUVEC | 132.1 | 290.3 | 579.3 | 250.2 | 1044.0 | 218.5 |
| BEAS | 131.3 | 260.5 | 562.2 | 124.7 | 1111.0 | 232.3 |

LTX-gallicune-1 (I1-N11, I1K10) and LTX-gallicune-1D analogues (iln11, I1N11, i1k10, I1K10) showed no cytotoxicity against mammalian HUVEC, IMR 90 and Beas cells at high concentration.

The Therapeutic Index (TI) of the most active peptides was determined by comparing their antimicrobial and cytotoxic dose-effects. The TI was determined by dividing the IC50 value found with one peptide on the more sensitive normal human cells by the MIC value found with the same peptide on the Pseudomonas aeruginosa. Using this equation, TIs of LTX-gallicune-1 (I1-N11, I1K10) and LTX-gallicune-1D analogues (iln11, I1N11, i1k10, I1K10) were found between 116.7 and 1111 on the three cell lines (Table 6).

**Protease stability of LTX-gallicune-1-(11N11) peptide and analogues**

The stability of engineered peptides of the invention was assessed by evaluating their resistance to proteases such as pepsin, trypsin and chemotrypsin. Results are summarized in Table 9 below.

**Table 9: MIC ($\mu$M) for engineered peptides of the invention treated or not with proteases against *Pseudomonas aeruginosa***

| Peptides | Control | Pepsin | Trypsin | Chymotrypsin | Serum4h | Serum24h |
|---|---|---|---|---|---|---|
| I1N11 | 6.25 | >50 | >50 | >50 | >50 | >50 |
| i1(D)k10(D) | 12.5 | >50 | >50 | >50 | >50 | 50 |
| I1K10 | 0.75 | >50 | >50 | >50 | >50 | >50 |
| i1(D)n11(D) | 3.125 | >50 | >50 | >50 | >50 | 25 |
| (I1K10)D | 1.5 | 1.5 | 1.5 | 1.5 | 1.5 | 0.75 |
| (I1N11)D | 25 | 25 | 25 | 25 | 25 | 12.5 |
| Control without AMP | - | >50 | >50 | >50 | >50 | 25 |

Stability assay demonstrated that only peptides LTX-gallicune-1D- (I1N11) and LTX-gallicune-1D-(I1K10) were not affected by proteases and human serum, demonstrating that they can be used in vivo without inactivation by digestive intestinal enzymes or human serum.

**Antiproliferative activity of LTX-gallicune-1-(11N11) peptides**

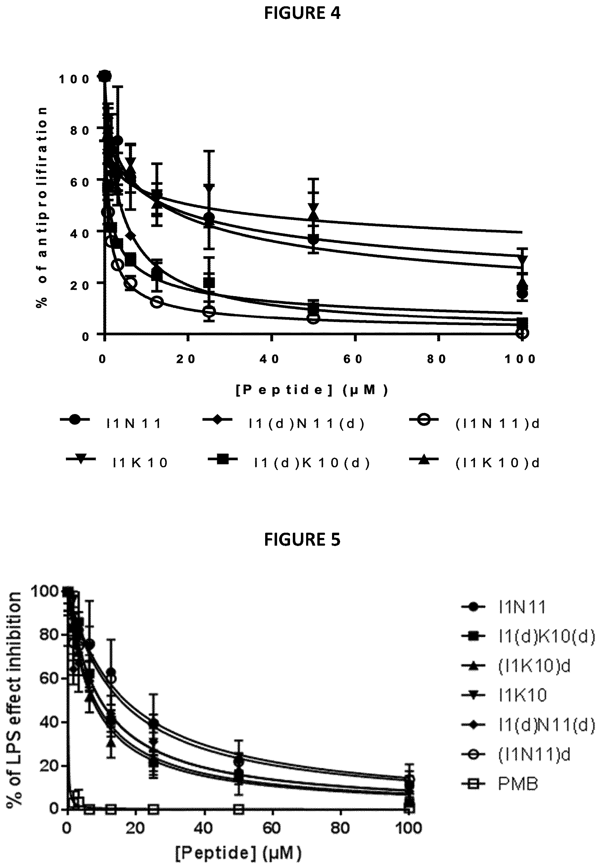

The antiproliferative activity of LTX-gallicune-1-(I1N11) peptides against various human cancer cells was examined by resazurin assay (Fig 4 and Table 10). Cancer cells tested were: MiaPaCa-2, PC-3 and MDA-MB-231 corresponding to pancreas, prostate and breast cancers, respectively. As shown in figure 4, the LTX-gallicune1 peptides altered proliferative capacity of the MiaPaCa-2 cell line. Activity against PC-3 and MDA-MB-231 were lower suggesting a selective action of gallicune peptides on pancreatic cancer cells. No antiproliferative activity was shown against HUVEC (data not shown).

**Table 10: Antiproliferative effect of gallicune peptides on cancer cell proliferation.**

| Cells | I1N11 | I1(d)K10(d) | (I1K10)d | I1K10 | I1(d)N11(d) | (I1N11)d |
|---|---|---|---|---|---|---|
| MiaPaCa-2 | 9.773 | 2.362 | 13.4 | 23.02 | 4.61 | 6.513 |
| PC-3 | 50.78 | 56.42 | 39.29 | 46.13 | 32.27 | 33.73 |
| MDA-MB-231 | 119.5 | 104.5 | 61.74 | 120.8 | 75.61 | 56.16 |

| Cells | Doxo | Col |
|---|---|---|
| MiaPaCa-2 | 0.1897 | 0.00152 |
| PC-3 | 0.315 | 0.0006994 |
| MDA-MB-231 | 0.07983 | 0.009012 |

Human cancer cells were seeded at 5,000 cells/well, were treated with increasing concentration of gallicune peptides and were left to proliferate for 48 h. After that, number of living cells was determined as explained previously. IC50 were determined from Fig 4 and are expressed in $\mu$M. Doxorubicine and colchicine were used as positive controls.

**Antiinflammatory effect of LTX-gallicune-1-(11N11) peptides**

Using the HeLa TLR4/IL-8 Renilla Luciferase cell line, we found that all gallicune peptide analogs inhibit the effect of the lipopolysaccharide (LPS) from *Pseudomonas aeruginosa* (from Sigma Aldrich) in a dose-dependent manner with IC50 ranging from 7.2 $\mu$M (LTX-gallicune-1-(i1k10) enantio to 16.8 $\mu$M (LTX-gallicune-1-(I11N11) (see Table 11 and Fig 5). For comparison, PMB (polymyxin B) was used as positive control and gave an IC50 of 0.057 $\mu$M.

**Table 11: Anti-inflammatory effect of Gallicune peptides on LPS effect from *Pseudomonas aeruginosa* on HeLa TLR4/IL-8 Renilla Luciferase cell line.**

| | I1N11 | I1(d)K10(d) | (I1K10)d | I1K10 | I1(d)N11(d) | (I1N11)d | PMB |
|---|---|---|---|---|---|---|---|
| IC50 | 16.86 | 9.481 | 7.242 | 9.552 | 7.876 | 15.37 | 0.05759 |

HeLa TLR4/IL-8 Renilla Luciferase reporter cells were treated with 10 ng/mL of LPS from P. aeruginosa in the presence of increasing concentrations of Gallucine peptides or polymyxin B (PMB) as positive control. After 6 h incubation, the induction of the expression of Renilla luciferase was measured as indicator of inflammation. Inhibitory concentrations 50 (IC50) corresponding to the concentration of peptide inhibiting 50 % of the LPS effect were calculated from Fig 5 and are expressed in $\mu$M.

**Membrane permeabilization assay**

The mechanism of action of peptides was first studied on whole bacteria. Most cationic antimicrobial peptides induce bacterial death by impacting the integrity of the cytoplasmic membrane. The permeabilizing capacity of the peptides of the invention was therefore evaluated.

This study was performed by comparison with the effect of Cetrimonium Bromide (CTAB). CTAB is a cationic detergent inducing membrane permeabilization. The interaction of PAMs with bacterial membranes was investigated using a Propidium Iodide (PI) assay.

The experiments were carried out on the same bacterial strains as those used initially for the determination of the MIC. These are two Gram[+] strains and two Gram[-] strains, one of which is pathogenic.

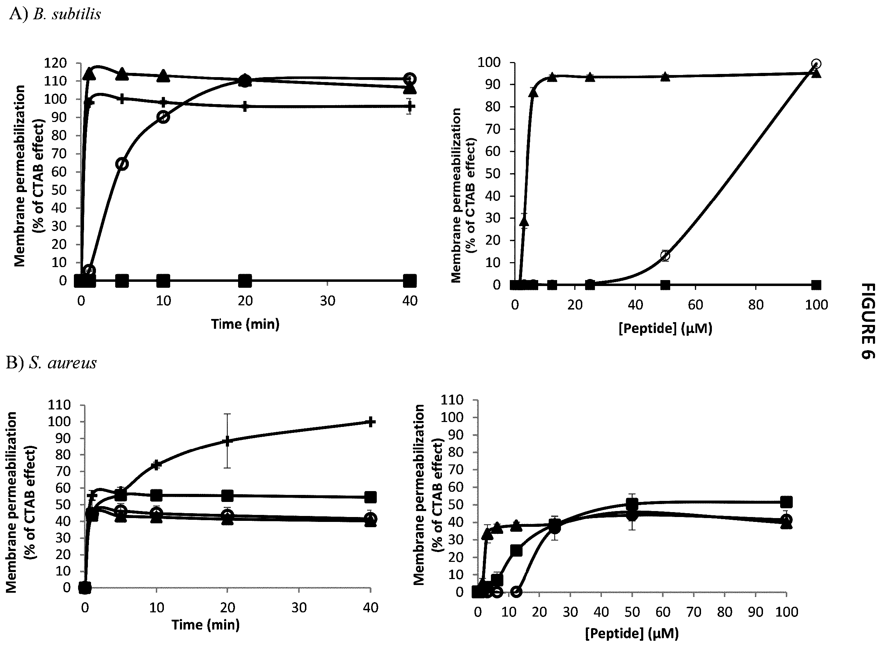

For each strain, the kinetics of membrane permeabilization were measured at different doses of peptides at 37 ° C (Figure 6A-D).

The results show that the antibacterial effect of the peptides is related to their ability to permeabilize the bacterial membranes. They induce an increase in membrane permeabilization in a dose-dependent manner.

The permeabilizing effect is very fast against *B.subtilis*. At 100 $\mu$M, all the peptides induce a permeabilizing effect which reaches a plateau between 40 and 50% of the standardized effect of the CTAB after one minute. Likewise, after 40 minutes of incubation, all the peptides induce a membrane permeabilization which reaches a plateau between 40 and 50% of the CTAB effect from 25 $\mu$M. The peptide Gallicus-CP, is the most active with a maximum of permeabilization reached from 6 $\mu$M, LTX-gallicune-1-(I1N11) and LTX-gallicune-2-(R1W8) between 25 and 50 $\mu$M with a lower permeabilizing effect at low doses for LTX-gallicune-2-(R1W8). In addition, Gallicus-CP is permeabilizing as early as 1.5 $\mu$M, LTX-gallicune-1-(I1N11) as early as 3 $\mu$M and LTX-gallicune-2-(R1W8) as between 12.5 and 25 $\mu$M. The permeabilizing doses correspond to their MICs.

Similarly, the permeabilization results obtained on *E. coli* are in agreement with the MIC of each peptide. Indeed, R1W8, which does not inhibit the growth of *E. coli,* does not permeabilize its membrane. After 40 minutes of incubation at 100 $\mu$M, the permeabilizing effect is negligible. Moreover, the antibacterial activity of the peptide Gallicus-CP against *E. coli* seems to be correlated with its low permeabilizing effect.

Similarly, LTX-gallicune-1-(I1N11) (E. coli (MIC >50 $\mu$M) induces 10% permeabilization at 100 $\mu$M.

LTX-gallicune-1-(I1N11) is 8 times more permeabilizing against P. aeruginosa than against *E. coli*. At 100 $\mu$M, it disrupts the integrity of the membrane from the first minute of incubation. A plateau at 80% of the standardized effect of CTAB is reached between 10 and 20 minutes. After 40 minutes of exposure, 6 $\mu$M are sufficient to induce permeabilization. Its dose-dependent action is similar to that of the Gallicus-CP peptide. The peptide LTX-gallicune-2-(R1W8) weakly permeabilizes the P. aeruginosa membrane with comparable kinetics and doses. A start of permeabilization is induced from 50 $\mu$M after 40 minutes of incubation, to reach 20 to 30% of the effect of CTAB at 100 $\mu$M. These results are in agreement with the activity tests against *P. aeruginosa.*

Finally, the permeabilization of the peptides was also performed on *S. aureus.* Except for LTX-gallicune-1-(I1N11), the effect of peptides on the cytoplasmic membrane of S. aureus is comparable to that against B. subtilis. In general, however, it is slower and requires more product. After 40 minutes of incubation, the I1L12 peptide is the most active with a maximum of permeabilization reached as early as 6 $\mu$M. 100% permeabilization is induced from the first minute of incubation with 100 $\mu$M I1L12. Although the process is slower for LTX-gallicune-2-(R1W8), this same plateau is also reached quickly (between 10 and 20 minutes of incubation). As for the peptide LTX-gallicune-1-(I1N11), it does not permeabilize the membrane of *S. aureus,* justifying its inactivity to 100 $\mu$M. All of these permeabilization results are consistent with those of MIC.

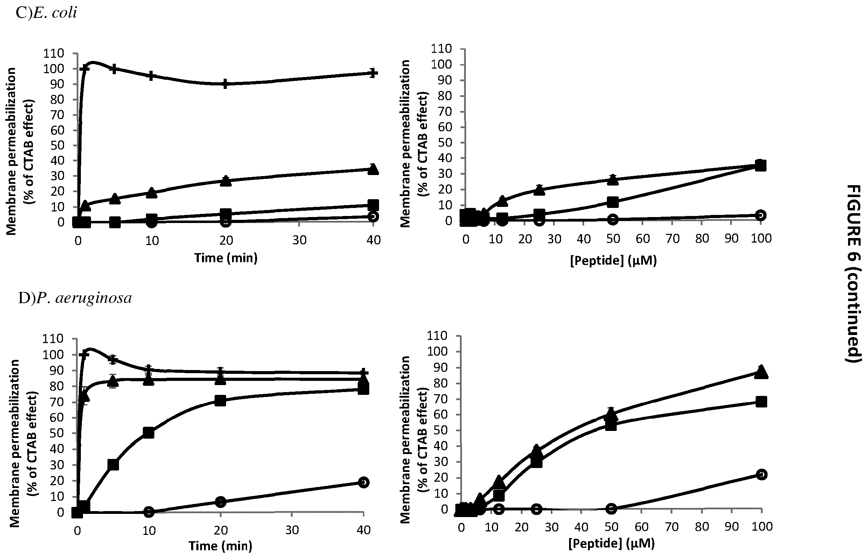

The permeabilizing effect of the peptides is correlated with their antibacterial activity. The peptides Gallicus-CP and LTX-gallicune-2-(R1W8) mainly active against Gram$^+$ strains have a permeabilizing effect in a few minutes on these strains and at doses comparable to their MIC (between 3 and 25 $\mu$M). On the other hand, they are little or not active against Gram s- and this seems to be explained by a low permeabilizing capacity even at 100 $\mu$M (Fig. 6).

## Selection for resistant bacteria

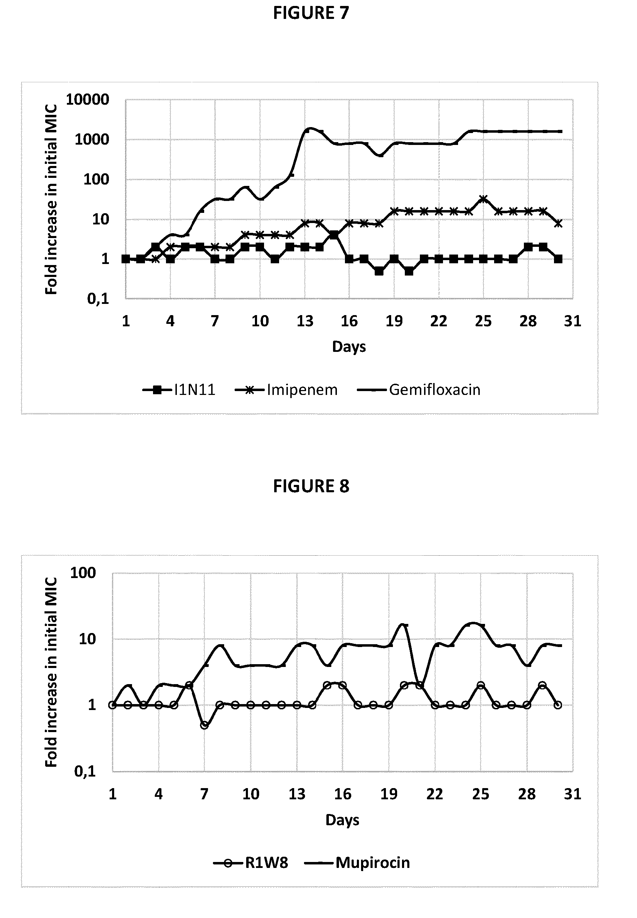

The investigation of whether a reference strains of *P. aeruginosa* and *B. subtilis* would evolve resistance after multiple exposures to gallicune peptides LTX-gallicune (I1-N11) and LTX-gallicune-2-(R1W8) was done over a period of 30 days. The MICs of the peptides against bacteria were not changed during the 30 days of testing. In contrast, the MIC of Imipenem and Gemifloxacin against *P. aeruginosa* and of Mupirocin against *B. subtilis* were increasing after exposure to these antibiotics for 10 days, demonstrating that these conventional antibiotics were quickly causing resistance acquisition (Fig. 7). This result shows that, contrarily to conventional antibiotics, in addition to be active on antibiotic-resistant strains, gallicune peptides do not cause the selection of resistant bacteria.

## Claims

1. An engineered peptide consisting of the amino acid sequence ILX$_1$X$_2$X$_3$X$_4$X$_5$LLX$_6$NL (SEQ ID NO:1), wherein:

   X$_1$ is absent or is an amino acid selected from the group consisting of S, K and W,
   X$_2$ is absent or is an amino acid selected from the group consisting of A and L,
   X$_3$ is an amino acid selected from the group consisting of I and W,
   X$_4$ is an amino acid selected from the group consisting of L and K,
   X$_5$ is an amino acid selected from the group consisting of G or K, and
   X$_6$ is an amino acid selected from the group consisting of K and R,
   and the functional derivatives or salts thereof,
   wherein the functional derivatives are selected from diastereomeric peptides, all-D-peptides, retropeptides, and peptides in which the sequence of SEQ ID NO:1 is shortened by 1 to 4 amino acids at the N-terminal and /or C-terminal end with the proviso that the peptide has a size comprised between 7 and 10 amino acids.

2. The engineered peptide of claim 1, wherein X$_1$ is an amino acid selected from the group consisting of S and K, X$_2$ is A, X$_3$ is I, X$_4$ is an amino acid selected from the group consisting of L and K, X$_5$ is an amino acid selected from the group consisting of G and K, X$_6$ is K.

3. The engineered peptide of claim 1, wherein $X_1$ is W, $X_2$ is L, $X_3$ is W, $X_4$ is L, $X_5$ is G and $X_6$ is R.

4. The engineered polypeptide of claim 1, consisting of an amino acid sequence selected from the group consisting of sequences of:

   - SEQ ID NO: 3,
   - SEQ ID NO: 4,
   - SEQ ID NO: 5,
   - SEQ ID NO: 6,
   - SEQ ID NO: 7,
   - SEQ ID NO: 8,
   - SEQ ID NO: 10,
   - SEQ ID NO: 11,
   - SEQ ID NO: 12,
   - SEQ ID NO: 13, and
   - SEQ ID NO: 14.

5. The engineered polypeptide of claim 1, consisting of an amino acid sequence selected from the group consisting of sequences of SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO:24, SEQ ID NO: 25, SEQ ID NO: 26, SEQ ID NO: 27 and SEQ ID NO: 28.

6. A nucleic acid encoding for an engineered peptide as defined in anyone of claims 1 to 5.

7. An expression cassette comprising a nucleic acid as defined in claim 6.

8. An expression vector comprising a nucleic acid as defined in claim 6 or an expression cassette as defined in claim 7.

9. A host cell comprising a nucleic acid as defined in claim 6, an expression cassette as defined in claim 7 or an expression vector as defined in claim 8.

10. A cosmetic composition comprising at least one engineered peptide as defined in any one of claims 1 to 5, and a cosmetically acceptable support and/or excipient.

11. A pharmaceutical composition comprising at least one engineered peptide as defined in any one of claims 1 to 5, and a pharmaceutically acceptable support and/or excipient.

12. An engineered peptide as defined in any one of claims 1 to 5 for use as medicament.

13. An engineered peptide as defined in any one of claims 1 to 5 for use in the treatment of a condition caused by a bacterium or a fungus.

14. *In vitro* use of an engineered peptide as defined in any one of claims 1 to 5 as disinfectant or preservative agent.

15. Medical device or implant comprising a body having at least one surface coated with or including an engineered peptide as defined in any one of claims 1 to 5.

**Patentansprüche**

1. Design-Peptid, bestehend aus der Aminosäurensequenz $ILX_1X_2X_3X_4X_5LLX_6NL$ (SEQ ID NO: 1), in der:

   $X_1$ fehlt oder eine Aminosäure, gewählt aus der Gruppe bestehend aus S, K und W, ist,
   $X_2$ fehlt oder eine Aminosäure, gewählt aus der Gruppe bestehend aus A und L, ist,
   $X_3$ eine Aminosäure, gewählt aus der Gruppe bestehend aus I und W, ist,
   $X_4$ eine Aminosäure, gewählt aus der Gruppe bestehend aus L und K, ist,
   $X_5$ eine Aminosäure, gewählt aus der Gruppe bestehend aus G und K, ist und
   $X_6$ eine Aminosäure, gewählt aus der Gruppe bestehend aus K und R, ist,

und die funktionsfähigen Derivate oder Salze davon,

wobei die funktionsfähigen Derivate unter diastereomeren Peptiden, all-D-Peptiden, Retropeptiden und Peptiden, in denen die Sequenz von SEQ ID NO: 1 um 1 bis 4 Aminosäuren am N-Terminus und/oder am C-Terminus gekürzt wurde, sofern das Peptid eine Länge von 7 bis 10 Aminosäuren aufweist.

2. Design-Peptid nach Patentanspruch 1, in dem $X_1$ eine Aminosäure, gewählt aus der Gruppe bestehend aus S und K, ist, $X_2$ A ist, $X_3$ I ist, $X_4$ eine Aminosäure, gewählt aus der Gruppe bestehend aus L und K, ist, $X_5$ eine Aminosäure, gewählt aus der Gruppe bestehend aus G und K, ist, $X_6$ K ist.

3. Design-Peptid nach Patentanspruch 1, in dem $X_1$ W ist, $X_2$ L ist, $X_3$ W ist, $X_4$ L ist, $X_5$ G ist und $X_6$ R ist.

4. Design-Polypeptid nach Patentanspruch 1, bestehend aus einer Aminosäurensequenz, gewählt aus der Gruppe bestehend aus den Sequenzen von:

- SEQ ID NO: 3,
- SEQ ID NO: 4,
- SEQ ID NO: 5,
- SEQ ID NO: 6,
- SEQ ID NO: 7,
- SEQ ID NO: 8,
- SEQ ID NO: 10,
- SEQ ID NO: 11,
- SEQ ID NO: 12,
- SEQ ID NO: 13, und
- SEQ ID NO: 14.

5. Design-Polypeptid nach Patentanspruch 1, bestehend aus einer Aminosäurensequenz, gewählt aus der Gruppe, bestehend aus den Sequenzen von SEQ ID NO: 15, SEQ ID NO: 16, SEQ ID NO: 17, SEQ ID NO: 18, SEQ ID NO: 19, SEQ ID NO: 20, SEQ ID NO: 21 SEQ ID NO: 22, SEQ ID NO: 23, SEQ ID NO: 24, SEQ ID NO: 25, SEQ ID NO: 26, SEQ ID NO: 27 und SEQ ID NO: 28.

6. Nukleinsäure, ein Design-Peptid nach irgendeinem der Patentansprüche 1 bis 5 codierend.

7. Expressionskassette, eine Nukleinsäure nach Patentanspruch 6 umfassend.

8. Expressionsvektor, eine Nukleinsäure nach Patentanspruch 6 oder eine Expressionskassette nach Patentanspruch 7 umfassend.

9. Wirtszelle, eine Nukleinsäure nach Patentanspruch 6, eine Expressionskassette nach Patentanspruch 7 oder einen Expressionsvektor nach Patentanspruch 8 umfassend.

10. Kosmetik-Zubereitung, mindestens ein Design-Peptid nach irgendeinem der Patentansprüche 1 bis 5 enthaltend, und einen Kosmetik-verträglichen Trägerstoff und/oder Vehikel.

11. Pharmazeutische Zubereitung, mindestens ein Design-Peptid nach irgendeinem der Patentansprüche 1 bis 5 enthaltend, und einen pharmazeutisch verträglichen Trägerstoff und/oder Vehikel.

12. Design-Peptid nach irgendeinem der Patentansprüche 1 bis 5 zum Gebrauch als Arzneimittel.

13. Design-Peptid nach irgendeinem der Patentansprüche 1 bis 5 zum Gebrauch in der Behandlung einer durch ein Bakterium oder einen Pilz verursachten Erkrankung.

14. Gebrauch *in vitro* eines Design-Peptids nach irgendeinem der Patentansprüche 1 bis 5 als Desinfektionsmittel oder Konservierungsstoff.

15. Medizinische Vorrichtung oder Implantat, einen Körper umfassend, von dem mindestens eine Oberfläche mit einem Design-Peptid nach irgendeinem der Patentansprüche 1 bis 5 beschichtet ist oder dieses enthält.

**Revendications**

1. Un peptide fabriqué consistant en la séquence $ILX_1X_2X_3X_4X_5LLX_6NL$ (SEQ ID NO:1), dans laquelle :

   $X_1$ est absent ou est un acide aminé choisi dans le groupe constitué de S, K et W,
   $X_2$ est absent ou est un acide aminé choisi dans le groupe constitué de A et L,
   $X_3$ est un acide aminé choisi dans le groupe constitué de I et W,
   $X_4$ est un acide aminé choisi dans le groupe constitué de L et K,
   $X_5$ est un acide aminé choisi dans le groupe constitué de G ou K, et
   $X_6$ est un acide aminé choisi dans le groupe constitué de K et R,
   et les dérivés fonctionnels ou sels de celui-ci,
   dans lequel les dérivés fonctionnels sont choisis dans le groupe constitué des peptides diastéréoisomériques, des peptides tout-D, des rétropeptides, et des peptides dans lesquels la séquence de la SEQ ID NO:1 est raccourcie de 1 à 4 acides aminés à l'extrémité N-terminale et/ou C-terminale, étant entendu que le peptide a une taille comprise entre 7 et 10 acides aminés.

2. Le peptide fabriqué selon la revendication 1, dans lequel $X_1$ est un acide aminé choisi dans le groupe constitué de S et de K, $X_2$ est A, $X_3$ est I, $X_4$ est un acide aminé choisi dans le groupe constitué de L et K, $X_5$ est un acide aminé choisi dans le groupe constitué de G et K, $X_6$ est K.

3. Le peptide fabriqué selon la revendication 1, dans lequel $X_1$ est W, $X_2$ est L, $X_3$ est W, $X_4$ est L, $X_5$ est G et $X_6$ est R.

4. Le polypeptide fabriqué selon la revendication 1, consistant en une séquence d'acides aminés choisie dans le groupe constitué des séquences de :

   - SEQ ID NO: 3,
   - SEQ ID NO: 4,
   - SEQ ID NO: 5,
   - SEQ ID NO: 6,
   - SEQ ID NO: 7,
   - SEQ ID NO: 8,
   - SEQ ID NO: 10,
   - SEQ ID NO: 11,
   - SEQ ID NO: 12,
   - SEQ ID NO: 13, et
   - SEQ ID NO: 14.

5. Le polypeptide fabriqué selon la revendication 1, consistant en une séquence d'acides aminés choisie dans le groupe constitué des séquences SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO:24, SEQ ID NO: 25, SEQ ID NO: 26, SEQ ID NO: 27 et SEQ ID NO: 28.

6. Un acide nucléique codant pour un peptide fabriqué tel que défini à l'une quelconque des revendications 1 à 5.

7. Une cassette d'expression comprenant un acide nucléique tel que défini à la revendication 6.

8. Un vecteur d'expression comprenant un acide nucléique tel que défini à la revendication 6 ou une cassette d'expression telle que définie à la revendication 7.

9. Une cellule hôte comprenant un acide nucléique tel que défini à la revendication 6, une cassette d'expression telle que définie à la revendication 7 ou un vecteur d'expression tel que défini à la revendication 8.

10. Une composition cosmétique comprenant au moins un peptide fabriqué tel que défini à l'une quelconque des revendications 1 à 5, et un support et/ou un excipient cosmétiquement acceptable.

11. Une composition pharmaceutique comprenant au moins un peptide fabriqué tel que défini à l'une quelconque des revendications 1 à 5, et un support et/ou un excipient pharmaceutiquement acceptable.

12. Un peptide fabriqué tel que défini à l'une quelconque des revendications 1 à 5 pour une utilisation en tant que médicament.

13. Un peptide fabriqué tel que défini à l'une quelconque des revendications 1 à 5 pour une utilisation dans le traitement d'une infection causée par une bactérie ou un champignon.

14. Utilisation *in vitro* d'un peptide fabriqué tel que défini à l'une quelconque des revendications 1 à 5 en tant qu'agent désinfectant ou agent conservateur.

15. Un dispositif médical ou un implant comprenant un corps ayant au moins une surface revêtue de ou comprenant un peptide fabriqué tel que défini à l'une quelconque des revendications 1 à 5.

## FIGURE 1-A

## FIGURE 1-B

## FIGURE 2

## FIGURE 3

**FIGURE 4**

**FIGURE 5**

FIGURE 6

A) *B. subtilis*

B) *S. aureus*

# FIGURE 6 (continued)

C) *E. coli*

D) *P. aeruginosa*

## FIGURE 7

## FIGURE 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4889803 A
- US 5047335 A
- US 20160354294 A1
- WO 2005006938 A

**Non-patent literature cited in the description**

- **KONNO et al.** *Peptides*, November 2006, vol. 27 (11), 2624-31
- **GOMES et al.** *Biochim Biophys Acta*, January 2014, vol. 1840 (1), 170-83
- **VÁCLAV CEROVSKÝ et al.** New potent antimicrobial peptides from the venom of Polistinae wasps and their analogs. *Peptides*, June 2008, vol. 29 (6), 992-1003
- **YOUNT** ; **YEAMAN**. *Pharmacol. Rev.*, 2003, vol. 55, 27-55
- **SAMBROOK et al.** Molecular Cloning: a laboratory manual. Cold Spring Harbor, 2001
- **LAN-YING LEE** ; **STANTON B. GELVIN**. *T-DNA Binary Vectors and Systems Plant Physiol.*, February 2008, vol. 146 (2), 325-332
- Remington's Pharmaceutical Sciences. Mack Publishing Company, 1990
- Pharmaceutical Formulation Development of Peptides and Proteins. Taylor & Francis, 2000
- Handbook of Pharmaceutical Excipients. Pharmaceutical Press, 2000
- **MERRIFIELD B**. *Science*, 18 April 1986, vol. 232 (4748), 341-7
- **MABROUK et al.** *Biochim Biophys Acta*, October 2007, vol. 1768 (10), 2528-40
- **DI PASQUALE et al.** *Chem Phys Lipids.*, 2010, vol. 63 (2), 131-40
- **OLLEIK et al.** *European Journal of Medicinal Chemistry*, 01 March 2019, vol. 165, 133-141
- **OYAMA et al.** *Biofilms Microbiomes*, 01 December 2017, vol. 3, 33
- **BORIE et al.** *Eur J Med Chem.*, 2018, vol. 148, 306-313